(19) Europäisches Patentamt
European Patent Office
Office européen des brevets

(11) EP 3 188 690 B1

(12) **EUROPEAN PATENT SPECIFICATION**

(45) Date of publication and mention of the grant of the patent:
**23.10.2024 Bulletin 2024/43**

(21) Application number: **14815435.4**

(22) Date of filing: **29.10.2014**

(51) International Patent Classification (IPC):
***A61F 2/16*** *(2006.01)*

(52) Cooperative Patent Classification (CPC):
**A61F 2/1637; A61F 2/1645; A61F 2/1654;**
A61F 2002/1689; A61F 2250/0097

(86) International application number:
**PCT/IB2014/065686**

(87) International publication number:
**WO 2016/034925 (10.03.2016 Gazette 2016/10)**

(54) **INTRAOCULAR LENS CUSTOMIZED FOR ASTIGMATISM OR COMBINED ASTIGMATISM AND PRESBYOPIA**

FÜR WEITSICHTIGKEITSKORREKTUR ODER KOMBINIERTE ASTIGMATISMUS UND PRESBYOPIE ANGEPASSTE INTRAOKULARLINSE

LENTILLE INTRAOCULAIRE PERSONNALISÉE POUR UN ASTIGMATISME OU UNE PRESBYTIE ET UN ASTIGMATISME COMBINÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2014 IN 2796MU2014**

(43) Date of publication of application:
**12.07.2017 Bulletin 2017/28**

(73) Proprietor: **Dave, Jagrat Natavar**
**Vadodara, Gujarat 319440 (IN)**

(72) Inventors:
- **ARGAL, Sanjay Ram Swaroop**
**Gujarat 390008 (IN)**
- **HUSSAIN, Munavvar Tahir**
**Gujarat 390020 (IN)**

(74) Representative: **Patentanwälte Bauer Vorberg Kayser**
**Partnerschaft mbB**
**Goltsteinstraße 87**
**50968 Köln (DE)**

(56) References cited:
**EP-A2- 2 111 822**
**WO-A1-2011/024125**
**WO-A1-99/29266**
**US-A1- 2009 323 020**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 188 690 B1

## Description

### FIELD AND BACKGROUND OF THE INVENTION

The present invention, in some embodiments thereof, relates to an intraocular lens (IOL) customized for astigmatism or a combination of astigmatism and presbyopia or other eye conditions and, more particularly, but not exclusively, to such a lens that requires only straightforward alignment.

The human eye is a complex anatomical device, which facilitates interpretation of shapes, colors and dimensions of objects by processing the light they reflect or emit. Similarly to a camera, the eye is able to refract light and produce a focused image that can stimulate neural responses and provide the ability to see.

For the purpose of providing a self-contained document, the following is a description of the principle of operation of the mammalian eye, in general, and of the cornea in particular. Light from our surroundings enters our eye through the dioptric media --- cornea, lens, aqueous humour and vitreous body. Among these, the anterior part of the cornea accounts for providing nearly 2/3 of the refractive power, because it has a highly curved surface and high refractive index.

Light stimulates the photoreceptors on our retina to produce nerve impulses, which will travel along the optic nerve to the *visual cortex* of our brain. The image formed on the retina is real, inverted and smaller. However, on interpretation by the brain, the images will be upright.

The quality of the image depends on many factors including size, shape and length of the eye and the shape and transparency of the cornea and lens.

The iris regulates the amount of light admitted to the interior of the eye, the cornea and the lens focus the light rays from an object being viewed onto the retina which transmits the image of the object to the brain via the optic nerve. About 75 % of the focusing is provided by the cornea, with the other 25 % provided by the crystalline lens which may acquire variable focal lengths.

The cornea is the most anterior structure of the eye. Since it has to be transparent to allow light to enter the eye, there are no blood vessels in the cornea. The cornea is composed of collagen fibers packed together in an organized pattern, thereby providing the cornea its light transparent nature. The cornea has the highest concentration of nerve endings in the entire body, thus making it extremely sensitive to any kind of trauma.

The front view of the cornea is of an aspheric shape, where the vertical dimension is smaller than the horizontal dimension by about 1-2 %. The anterior is typically about 11.7 mm in diameter.

The quality of vision depends on many factors including the size and shape of the eye, and the transparency of the cornea and lens. When age or disease causes the lens to become less transparent, vision deteriorates because of the diminished light which can be transmitted to the retina. This deficiency in the lens of the eye is medically known as a cataract. A cataract is a clouding that develops in the crystalline lens or in its envelope, varying in degree from slight to complete opacity and obstructing the passage of light. Early in the development of age-related cataract the power of the lens may be increased, causing near-sightedness (myopia), and the gradual yellowing/opacification of the lens may reduce the perception of blue colors. Cataracts typically progress slowly to cause vision loss and are potentially blinding if untreated. The condition usually affects both eyes, but oftentimes one eye is affected earlier than the other.

An accepted treatment for this condition is surgical removal of the lens by making an incision (capsulotomy) into the capsule, and replacement of the lens function by an intraocular lens (IOL), which is typically a PMMA (polymethylemethacrylate) lens. Some patients suffer from irregular cornea (astigmatism). For these patients, a cylindrical correction is required after IOL implantation to get quality of vision.

In more detail, an intraocular lens (IOL) is a lens implanted in the eye used to treat cataracts. The most common type of IOL is the pseudophakic IOL. These are implanted during cataract surgery, after the cloudy crystalline lens, the cataract, has been removed. The pseudophakic IOL replaces the original crystalline lens, and provides the light focusing function originally undertaken by the crystalline lens. The second type of IOL, more commonly known as a phakic intraocular lens (PIOL), is a lens which is placed over the existing natural lens, and is used in refractive surgery to change the eye's optical power as a treatment for myopia or nearsightedness. IOLs usually consist of a small plastic lens with plastic side struts, called haptics, to hold the lens in place within the capsular bag inside the eye IOLs were traditionally made of an inflexible material, polymethyl methacrylate, (PMMA), although this has largely been superseded by the use of flexible materials. Most IOLs fitted today are fixed monofocal lenses matched to distance vision. However, other types are available, such as multifocal IOLs which provide the patient with multiple-focused vision at far and reading distance, and adaptive IOLs which provide the patient with limited visual accommodation.

Insertion of an intraocular lens for the treatment of cataracts is the most commonly performed eye surgical procedure. The procedure can be done under local anesthesia with the patient awake throughout the operation. The use of a flexible IOL enables the lens to be rolled for insertion into the capsule through a very small incision, thus avoiding the need for stitches, and this procedure usually takes less than 30 minutes in the hands of an experienced ophthalmologist. The recovery period is about 2-3 weeks.

IOL implantation carries several risks associated with eye surgeries, such as infection, loosening of the lens,

lens rotation, inflammation and night time halos. Lens rotation is particularly a problem if the lens is intended to deal with astigmatism.

Several methods are available to treat cataract in general but given the rotation issue, astigmatism reduces the number of options available, and the problem is only compounded when astigmatism and presbyopia etc occur together.

Typically, the power required of the intraocular lens is determined by the properties of the patient's eye which can include one or more refractive indices, curvature and /or distance. Any of the properties may be measured for particular patient, so that a selected power and overall size for the intraocular lens matches the power (Toric or Toric + Presbyopia) & size required for the particular eye to within a minimal tolerance, such as 0.25 or 0.50D depending of the patient's condition and other factors.

In some cases, a particular cornea may have a rotational asymmetry that imparts astigmatism onto light that is transmitted through it. The astigmatism degrades the vision of the eye and cannot be corrected by adjusting the power of the lens. In these cases the intraocular lens may provide additional correction if it has similar but opposite amount of astigmatism. But vision will only be improved if the IOL does not rotate within the eye after placement.

In practice, even without subsequent rotation, there are difficulties with an equal but opposite astigmatism correction. In particular, there may be some residual astigmatism left in the eye due to misalignment between the astigmatic axis of the cornea and the astigmatic axis of the intraocular lens.

Two types of eye surgery are known: extra-capsular (extracapsular cataract extraction, or ECCE) and intra-capsular (intracapsular cataract extraction, ICCE).

ECCE surgery consists of removing the lens but leaving the majority of the lens capsule intact. High frequency sound waves (phacoemulsification) can be used to break up the lens before extraction. ICCE surgery involves removing the entire lens of the eye, including the lens capsule.

Over the years, numerous types of IOLs have been developed for correcting vision deficiencies. Recent improvements in intraocular technology now provide a soft foldable lens made of silicon and acrylics. These lenses are popular because they can be inserted in a folded state through a small incision.

Generally, IOL lenses operate according to two basic optical principles: refraction and diffraction.

As mentioned, a typical IOL is manufactured from polymethyl methacrylate, has a diameter of about 5-7 mm, and is supported in the eye by the spring force of the flexible loops called haptics. Other materials are also used, and there are a variety of lens style and haptic designs.

Multifocal lens has more than one point of focus. A bifocal, which is a type of multifocal, has two points of focus, one at distance and the other at near. In multifocal IOL the aim is to increase the range of distinct vision and hence to reduce the dependence on additional spectacle corrections. Rigid lenses that have two or more optical powers are used to divide the incident light between axially separated images.

Overall image quality is affected by the number of lens powers, and the image quality of the focused component itself.

One type of multifocal IOL is diffractive multifocal IOL. A pair of diffraction orders is used to provide two lens powers simultaneously by using a rigid implant. One power is used for distance vision and the other power is used for near vision. In both cases defocused light is also incident on the retina, but the human visual system is tolerant of contrast-related image variations and this does not appear to be a problem for most patients. The diffractive design utilizes the full aperture and is tolerant of pupil size variations and modest decentration.

Generally, a diffractive lens consists of any number of annular lens zones of equal area. Between adjacent zones optical steps are provided with associated path length differences which usually are absolutely smaller than a design wavelength. The area or size of the zones determines the separation between the diffractive powers of the lens; this separation increases with decreasing zone area. The optical path difference determines the relative peak intensities of the various diffractive powers. For example, when the optical path difference equals half the wavelength there are two principal diffractive powers, the zeroth and the first order diffractive power. For absolute path differences which are smaller than half the wavelength, the zeroth order power is dominant, while for optical path differences which are of order of one wavelength the first diffractive order power is dominant.

The majority of ophthalmic lenses, including IOLs currently used are of a Monofocal Design. Most patients receiving an IOL still require glasses for clear distance and near vision with a target of-1D error due to surgical and measurement errors.

Various multifocal ophthalmic lens designs are currently under investigation and these designs generally fall into one of two categories, refractive lenses and diffractive lenses. Diffractive lenses use nearly periodic microscopic structures on the lens to diffract light into several directions simultaneously. This is similar to a diffraction grating and the multiple diffraction orders focus the light into various images corresponding to different focal lengths of the lens. Diffractive multifocal contact lenses and IOLs are more fully discussed in the following documents: US Patent 4881805 to Cohen, US Patent 5089023 to Swanson, US Patent 5699142 to Chun-Shen Lee (Alcon Restor), US Patent 534447 to Swanson, US Patent 7377641 to Patricia Ann Piers, European Patent 1194797B1(Acritec AcriLisa), U.S. Pat. Nos.

5,076,684, 5,116,111 (Simpson, et al.), U.S. Pat. No. 5,129,718 (Futhey, et at.) and U.S. Pat. Nos. 4,637,697, 4,641,934 and 4,655,565 (Freeman).

While a diffractive IOL may have a number of focal lengths, generally, IOLs with only two focal lengths (far and near) are the most common. As with any simultaneous vision multifocal lens, a defocused image (or images) is superimposed on the focused component because of the second lens power, but the defocused image is rarely observed by the user, who concentrates on the detail of interest. Under certain circumstances (for example, at night), the pupil diameter of the user can expand to 4 to 5 mm or more, and a discrete distant light source (e.g., automobile headlights or street lights) can appear to be surrounded by a "Halo" or "Rings" and Scattering of light through a non-transition area of each diffractive zone. A significant component of the halo is caused by the light that is directed to the near image which becomes defocused at the retina. The visibility of the halo is affected by the diameter of the lens region directing light to the near image, the proportion of total energy directed to the near image and the overall imaging aberrations of the eye.

In U.S. Pat. No. 4,881,805, Cohen suggests that the intensity of light traveling through a diffractive lens can be varied by reducing the echelette depth at the lens periphery, thus reducing glare.

Cohen's theory states that the glare results from the depth of the steps at the diffractive zone boundaries may be more applicable to contact lenses than intraocular lenses. Contact lenses generally move on the eye and the grooves can become filled with debris (tears).

In addition, the additive power of the contact lenses generally is less than that of intraocular lenses, which puts the defocused image more in focus, and also the patient's natural residual accommodation may alter the visibility of glare or halos.

The present inventor thus worked on providing a diffractive, multifocal IOL that minimizes glare or halos.

A Diffractive Lens design provides two optical powers through natural optical diffraction & interference phenomena, by operating on the simultaneous vision principle, where one image is in focus on the retina and the second image is formed at a different Axial location and is thus highly defocused on the Retina.

Given a step depth smaller than one wavelength, say 1/2 $\lambda$, bifocality can be achieved.

In manufacturing a bifocal lens, a diffractive monofocal structure may be provided at the optic rim to increase the refractive power of the lens, thus providing diffractive near vision and asymmetric light distribution between the far and near focus.

With a step height of less than 2$\mu$m or one wavelength of light, approx 30 annular rings can be formed within a 6mm optic diameter. The designed wavelength may be $\lambda$ =555nm.

Also known are lenses which are based on refractive principles. Such refractive lenses typically include concentric zones of differing power.

A known IOL lens for use in the case of astigmatism provides an equal and opposite amount of astigmatism as the cornea. If this lens were to be implanted in the eye with its astigmatism precisely oriented to that of the cornea, then corneal astigmatism would be completely or almost completely neutralized. However, there is usually an angular error in the orientation that arises during the surgical procedure. This error may be limited in practice by skilled surgeons, although less skilled surgeons may end up with refractive surprise due orientation error may be more than 30 degree.

Consider a cornea that has 4 diopters of astigmatism and a lens that has 4 diopters of the astigmatism. If the lens is now implanted with an angular error of 5 degrees, which is in fact a rather tight tolerance for a surgeon, then the residual astigmatism is 2Dsin5° = 0.70D. However, the typical threshold for astigmatism is 0.25D, so that only if the light reaching the retina has less than 0.25D astigmatism then the astigmatism does not significantly degrade the vision of the eye. It is therefore clear that a lens that has to be aligned so exactly by a surgeon is not an effective treatment when astigmatism is present.

Relevant prior art in this respect includes the following patent applications: US 2009/0323020A1 to Zhao et al, which discloses an intraocular lens for correcting or reducing the astigmatism of a cornea includes an optical element that has optical properties and characteristics that make it tolerant of rotational misalignment, when compared to a comparable lens having a uniform astigmatism orientation across its entire optical element, leading to more relaxed tolerances for a surgeon that implants the lens. The optical element of the toric ophthalmic lens has meridians associated therewith, including a high power meridian and a low power meridian orthogonal to the high power meridian. The optical element has at least one radially modulated meridian along which power monotonically varies with increasing radial position.

US 3722986 to Tagnon discusses a high toric power ophthalmic lens, and US 2008/0288066A1 discloses a "piggyback" cylindrical (toric) intraocular lens for placement in front of an accommodating or standard intraocular lens that is already in the capsular bag of the eye. This additional lens is placed in the sulcus, which leaves a significant space between the two lenses, particularly if the lens in the capsular bag is vaulted backwards.

US Patent Application No. 2009/0323020A1 discloses an intraocular lens for correcting or reducing the astigmatism of a cornea includes an optical element that has optical properties and characteristics that make it tolerant of rotational misalignment, when compared to a comparable lens having a uniform astigmatism orientation across its entire

optical element, leading to more relaxed tolerances for a surgeon that implants the lens. The optical element of the toric ophthalmic lens has meridians associated therewith, including a high power meridian and a low power meridian orthogonal to the high power meridian. The optical element has at least one radially modulated meridian along which power monotonically varies with increasing radial position.

EP2111822 A2 discloses an intraocular lens (IOL) with a toric optic for the correction of astigmatism, with on one side of a lens a most curved meridian with a most curved lens curvature and a flattest meridian with a lens curvature which is flatter than the most curved lens curvature, wherein an effective optical zone in the most curved meridian is larger or essentially equal to the effective optical zone in the flattest meridian.

## SUMMARY OF THE INVENTION

The invention relates to an intraocular lens as defined in claim 1 and to a method of forming an intraocular lens as defined in claim 15.

The present embodiments build a lens specifically for the astigmatism of the particular eye, and then build alignment into the shape of the lens.

An intraocular lens comprises a lens body customized for astigmatism in a particular eye. For the customization to work, the astigmatism in the lens has to be aligned with astigmatism in the eye. Thus the intraocular lens comprises four protrusions arranged outwardly around the lens body to form the four corners of a substantially rectangular profile. The profile locks the intraocular lens into a position of rotational stability when inserted into a capsular bag, thus preventing rotation of the intraocular lens, which would otherwise tend to occur both immediately after insertion into the capsular bag and later on during growth, shrinkage or other change to the capsular bag.

According to an aspect of some embodiments of the present invention there is provided an intraocular lens comprising:

a lens body customized for astigmatism in a particular eye, the customization requiring an alignment following insertion within the eye;
a plurality of protrusions arranged outwardly around the lens body to form a substantially rectangular profile, thereby to lock the intraocular lens into a position of rotational stability when inserted into a capsular bag, thus to prevent rotation of the intraocular lens after insertion into the capsular bag. The aspect is characterized in that the intraocular lens comprises a cylindrical profile for treatment of astigmatism.

In an embodiment, the protrusions comprise protrusions of haptic pads attached to the lens body.

In an embodiment, the protrusions form rounded corners of a rectangle around the lens body.

In an embodiment, the haptic pads are hinged to the lens body.

In an embodiment, the haptic pads comprise resilient material.

In an embodiment, the haptic pads comprise a cross-sectional profile which is thinner than that of the lens body.

An embodiment may comprise orientation markers to distinguish different orientations of the intraocular lens.

An embodiment may comprise axis markers to distinguish an optical axis of the intraocular lens.

The axis markers may comprise two groups of three holes respectively at different ends of the axis. Additionally and optionally, the groups may be distanced from an edge of the lens body by a predetermined margin.

The lens may comprise an overall length along a diagonal of substantially rectangular shape, the overall length being customized according to an estimated size of a respective capsular bag.

The lens may have a cylindrical profile to treat astigmatism.

The lens may have a toric profile to treat astigmatism.

The lens may have an aspherical profile to treat astigmatism.

The astigmatism of the lens may be aligned with an optical axis of the lens and the optical axis is marked with axis markers for alignment with the vertical or horizontal axis of the eye, with the aim of designing the astigmatism on the lens to line up with and cancel out the astigmatism of the eye.

The IOL may further include a diffractive lens.

In an embodiment, the diffractive lens provides a near focus and a far focus, thereby to provide bifocal intraocular lens.

The lens body may have two opposite sides, wherein a first side has an aspheric profile and a diffractive pattern formed thereon, and the second side may have a toric profile.

In an embodiment, the toric profile is devoid of spherical aberration and a cylinder value characterizing the lens body may be of at least 1 diopter.

In an embodiment, the diffractive pattern comprises a plurality of concentric annular zones having surfaces separated by slanted steps having surfaces, wherein the surfaces of the concentric zones effect both diffraction and refraction of incident light, while the surfaces of the steps are substantially devoid of any diffractive or refractive power.

In an embodiment, each step of the lens is characterized by a radius, a slope and a height, and wherein the slope and the height are at least non-increasing functions of the radius.
In an embodiment, the haptic pads are foldable.
In an embodiment, the lens body is made of biocompatible material.
In an embodiment, the lens body comprises a hydrophilic acrylic material.
In an embodiment, the lens body comprises a polymeric or co-polymeric composition, and at least one curcuminoid compound incorporated in or on the polymeric or co-polymeric composition and/or the lens body.
In an embodiment, the lens body comprises a co-polymeric composition having a polymeric backbone composed of a plurality of backbone units covalently linked to one another, the backbone units being derived from a pre-polymerization mixture of monomers.
In an embodiment, the lens body comprises a polymeric or co-polymeric composition derived from a pre-polymerization mixture of monomers which comprises at least 50 weight percents acrylate monomers.
In a second aspect of the present invention there is provided an intraocular lens comprising:

a lens body customized for astigmatism in a particular eye, the customization requiring an alignment following insertion within the eye;
a plurality of protrusions arranged outwardly around the lens body to form a substantially rectangular profile, thereby to lock said intraocular lens into a position of rotational stability when inserted into a capsular bag, thus to prevent rotation of said intraocular lens after insertion into said capsular bag, the intraocular lens characterized by further comprising axis markers to distinguish an optical axis of said intraocular lens, wherein said axis markers comprise two groups of three holes respectively at different ends of said axis, said groups distanced from an edge of said lens body by a predetermined margin.

According to a third aspect of the present invention there is provided a method of forming an intraocular lens, comprising:

obtaining an estimate of a length of a capsular bag of an eye requiring the intraocular lens;
forming a lens body having a first side and a second side, customizing the lens with astigmatism aligned along an optical axis to match an astigmatism of the eye; and
providing haptic pads around the lens body, the haptic pads being shaped to provide a substantially rectangular profile to surround the optical axis, the optical axis being aligned along the rectangular profile, and the estimate of a length being used to determine a size of the intraocular lens by defining a diagonal of the rectangular profile.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

## BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
In the drawings:

FIG. 1 is a simplified diagram illustrating a cross-section of an IOL according to a first embodiment of the present invention;
FIG. 2 is a simplified lateral view of the IOL of Fig. 1;
FIG. 3 is a simplified diagram illustrating a cross-section of an IOL according to a second embodiment of the present invention; and
FIG. 4 is a simplified lateral view of the lens of Fig. 3.

DESCRIPTION OF SPECIFIC EMBODIMENTS OF THE INVENTION

The present invention, in some embodiments thereof, relates to an IOL and, more particularly, but not exclusively, to an IOL that is designed for easy alignment when implanted in the eye.

The lens is customized for the particular eye with a particular alignment in mind. Then the lens is designed with a built-in alignment. That is to say the lens is built with haptic pads which specifically define a particular alignment and also with alignment guides built into the lens. The alignment guides may be a series of holes. The haptic pads may be flexible or springy and the lens may be injected into position in the eye.

In one method of implanting the IOL into the eye, injection from a capsule is used. Injection is through a small tube with the IOL partly folded, and the IOL unfolds into the capsular bag to automatically align due to the rectangular shape. The rectangular shape is rounded to prevent damage to the eye tissues. Guide markers allow for verification of the alignment of the optical axis, and also to ensure that the lens has not inadvertently flipped from back to front.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

When a ray of light moving in air and striking a surface of a light-transmissive substance at an angle $\alpha_1$ as measured from a normal to the surface, it is refracted into the substance at an angle which is determined by Snell's law, which is mathematically realized through the following equation:

$$n_A \sin \alpha_1 = n_S \sin \alpha_2$$

where ns is the index of refraction of the substance, $n_A$ is the index of refraction of the air, and $\alpha_2$ is the angle in which the ray is refracted into the substance. Similarly to $\alpha_1$, $\alpha_2$ is measured from a normal to the surface. A typical value of $n_A$ is about 1.

As used herein, the term "about" refers to ±10 %.

Another optical phenomenon is diffraction which is the slight bending of light as it passes around the edge of an object, or at an opening thereof. The amount of bending depends on the size of the wavelength of light compared to the size of the opening or edge. If the opening is much larger than the light's wavelength, the bending will be almost unnoticeable. However, if the two are closer in size or equal, the amount of bending is considerable, and easily seen with the naked eye.

Optical effects resulting from diffraction are produced through the interaction of light waves originating from different regions of the opening causing the diffraction. Illustratively, one can view this interaction as one of two types of interferences:

(i) a constructive interference when the crests of two waves combine to produce an amplified wave; and

(ii) a destructive interference when a crest of one wave and a trough of another wave combine, thus canceling each other.

A skilled artisan would, however, appreciate that there are many situations in which the interaction between the light waves is more complicated, e.g., when the light has a plurality of wavelengths.

One of the embodiments discussed below exploits the refraction and diffraction phenomena for devising a multifocal lens device. Typically, the lens device of the present embodiments has diffractive power for enabling near vision and refractive power for enabling far vision. In various exemplary embodiments of the invention the lens device also enable intermediate vision, as further detailed hereinunder.

Various embodiments of the present invention provide IOLs which include both toricity and asphericity to correct or mitigate corneal astigmatism and presbyopia. The toricity and asphericity is optionally and preferably on two separate surfaces. However, embodiments in which both toricity and asphericity are present on a single surface are not excluded from the scope of the present embodiments. A single asphericity may be presented for all cylinder meridians or a variable asphericity may be presented for different meridians. For examples, different degrees of asphericity may be used for the two primary meridians of the astigmatism. Embodiments disclosed herein may be useful for correcting or mitigating

other aberrations, such as coma, trefoil, tetrafoil, and the like. Correction of higher order aberrations are also contemplated.

In some embodiments of the present invention, a multifocal lens device is provided. The device has a lens body having an anterior surface and a posterior surface and one or more haptics coupled to the lens body. In various exemplary embodiments of the invention one of the posterior or anterior surfaces is shaped so that the lens body constitutes an aspheric lens, and in various exemplary embodiments of the invention one of the posterior or anterior surfaces is shaped so that the lens body constitutes a toric lens. For example, the anterior surface can be shaped so that the lens body constitutes an aspheric lens, and the posterior surface can be shaped so that the lens body constitutes a toric lens. Opposite configuration (the anterior surface constitutes a toric lens, and the posterior surface constitutes posterior an aspheric lens) are not excluded from the scope of the present invention.

Referring now to the drawings, Figure 1 illustrates a cross-sectional view of an IOL according to present embodiments of the invention.

In Fig. 1 lens body 100 comprises anterior side 102 and posterior side 104. Haptic pads 106 and 108 extend outwardly from the lens body 100 to give the overall IOL a square profile when viewed from the front or back of the lens. In Fig. 1, the anterior side 102 comprises a toric profile.

Reference is now made to Fig. 2, which illustrates the IOL of Fig. 1 viewed from the front or back of the lens, and showing the square profile of the IOL as a whole which is brought about by haptic pads 106 and 108.

The haptic pads have a width 110 which is substantially the same as the optical diameter 112 of the lens 100, and extending protrusions 114, 116, 118 and 120 at the corners. The extending protrusions are rounded so as not to cause damage to the eye tissue, but nevertheless give the IOL a substantially rectangular or squared profile to prevent rotation within the eye once implanted. That is to say the substantially rectangular profile effectively locks the lens within the capsular bag to provide stability against rotation and ensure that the astigmatism of the lens lines up against the astigmatism of the eye. In addition, optic - haptic junctions 122 and 124 are hinged and the haptic pad is thinner than the lens so that the haptic pads are compressed against the lens without pushing the lens out of position. The effect is to hold the lens more tightly in position between the two haptic pads, helping to fix the lens in a defined position. In particular, if the capsular bag contracts, the effect will be simply to hold the lens more tightly. In lenses of the current art, capsular bag contraction causes movement of the lens.

The optic -haptic junctions 122 and 124 and haptic protrusions 114 - 120 thus ensure predictable effective lens position, ELP. Due to the springiness, the ELP may be retained even in case of excess capsular bag contraction and may thus ensure constant refractive outcome under changing conditions.

Holes 126 and 128 are directional holes to ensure correct orientation or directionality of the lens in the capsular bag, and specifically to ensure that the lens is not flipped back to front. Holes 126 and 128 are located on opposite sides of a central axis at top and bottom of the lens respectively so that the top can be distinguished from the bottom, or if the lens is place transversally then they allow the right side to be distinguished from the left side. In the example illustrated, directional holes 126 and 128 are located on the left hand upper side of the lens and right hand lower side of the lens respectively. These holes allow the surgeon to distinguish up and down orientations of the lens. In particular, when introducing the lens through the cartridge tunnel into the eye during placement, there is some risk of the lens flipping over. The holes 126 and 128 ensure that such a flip is easily detectable by the surgeon.

Two aligned sets 130 and 132 of three holes each, extend along the vertical axis of the lens and can be used to ensure the alignment of the lens correctly along the optical axis of the eye to ensure that the astigmatism of the eye is matched by the astigmatism of the lens. The holes are located on the lens body 100 a small margin away from the haptic junctions 122 and 124 respectively.

Three holes are provided in each set to ensure that the holes are visible even in the case of smaller people and smaller eyes, where the margin from the haptic is relatively the same but in absolute terms smaller.

Depending on the patient, the axis marked by groups of holes 130 and 132 may be placed horizontally or vertically. The lens body includes a cylinder with customized toricity which is already aligned with the axis and thus the surgeon merely has to align the axis. In the prior art the surgeon had to carry out reference marking in a sitting position and then axis marking in the supine position, both steps having a standard deviation of about five degrees. Yet an error of just one degree leads to three percent loss in cylindrical power.

Profile 134 around the outer part of the lens is the toric profile of the lens. The toric profile may be customized for the individual patient and the individual eye according to the astigmatic axis of the particular patient.

Profile 136 is the inner part of the lens and comprises a non-toric profile that may also be customized for the individual patient. Profiles 134 and 136 are at right angles respectively.

Profiles 102 and 104 are the anterior and posterior surfaces respectively of the resulting customized monofocal Toric lens, the anterior profile 102 being the toric profile.

In order to customize the lens, the surgeon provides the size of what is known as white to white (WTW), the corneal diameter in which he wants to make the incision in order to insert the lens. The surgeon also indicates whether the incision is to be vertical or horizontal. Based on the WTW measure it is possible to estimate the size of the capsular

bag into which the lens is to be inserted. Based on the estimate, the lens and haptic pads can be sized to be large enough to grip the bag tightly enough to be stable and not rotate. On the other hand the lens and pads should be small enough not to cause any rupture or damage in general to the bag. The dimension that is selected based on the estimate is the overall length indicated by arrow 142, the length being the diagonal of the lens plus haptic pads. The length is selected to provide a fit into the capsular bag and to ensure that there is no rotation.

Overall length may vary between 10.00mm and 15.00mm in 0.25mm steps to ensure suitability for the maximum possible number of patients.

The present embodiments may ensure the rotational stability of the IOL, thanks to the square design of the haptic protrusions 114 - 120.

Each lens size is customized for the individual patient in order to keep capsular bag intact but at the same time to fit tightly within.

The present embodiments may provide an IOL which can be calculated for aphekia as a remedy of cataract & astigmatism, cataract & presbyopia, or cataract, astigmatism and presbyopia altogether.

The present embodiments may treat refractive error in psedophakic patients and provide multifocality with astigmatic correction, or for that matter independently of astigmatic correction.

Reference is now made to Fig. 3, which is a simplified diagram illustrating a cross-section of a Toric- Diffractive optical profile. Parts that are the same as in Fig. 1 are given the same reference numerals and are not described again except as needed for an understanding of the present embodiment.

In Fig. 3 lens body 100 comprises anterior side 138 and posterior side 140. Haptic pads 106 and 108 extend outwardly from the lens body 100 to give the overall IOL a square profile when viewed from the front or back of the lens. In Fig. 3, the anterior side 138 comprises a refractive profile and the posterior side 140 comprises a toric profile.

Reference is now made to Fig. 4, which illustrates the IOL of Fig. 3 viewed from the front or back of the lens. Parts that are the same as in Fig. 2 are given the same reference numerals and are not described again except as needed for an understanding of the present embodiments. Fig. 4 shows the square profile of the IOL as a whole which is brought about by haptic pads 106 and 108 as before.

Diffractive lens 144 is located in front of non-toric part 136 of the overall toric profile lens. The diffractive lens comprises small stepped rings arranged in an overall diffractive profile.

The combination of both a diffractive 138, 144, and a toric 140, structure provide a diffractive ophthalmic lens that can provide compensation for both spherical and astigmatic aberration while providing both a far-focus and a near-focus power to define a bi-focal lens.

The lenses are now considered in greater detail. Lens body 100 can be made of any material which is sufficiently transparent to visible light and which is suitable for optics. In various exemplary embodiments of the invention the lens body is made of biocompatible material, such as, but not limited to, hydrophilic acrylic material. In various exemplary embodiments of the invention the lens body or at least the haptic pads, are foldable.

As explained above, in the embodiment of Figs 3 and 4, first side 138 has an aspheric profile, and second side 140, also 104 of Fig. 1, have a toric profile.

The term "aspheric profile" is well known to those skilled in the art. To the extent that any further explanation may be required, this term is employed herein to refer to a radial profile of a surface that exhibits deviations from a spherical surface. Such deviations can be characterized, for example, as smoothly varying differences between the aspherical profile and a putative spherical profile that substantially coincides with the aspherical profile at the small radial distances from the apex of the profile.

From a mathematical standpoint, an aspheric profile is a type of a conic section. A conic section can be characterized by a parameter known as a conic constant *k,* wherein $k > -1$ corresponds to an ellipse ($k > -1$, $k \neq 0$) or a circle ($k \neq 0$), $k = -1$ corresponds to a parabola, and a $k < -1$ corresponds to a hyperbola.

For a given conic section *k*, the sag Z(s) of surface 138 at any point s, s being the radial distance from the optical axis of lens device 100, can be written as:

$$Z(s) = \frac{Cs^2}{1+\sqrt{1-(1+k)C^2s^2}} + A_4s^4 + A_6s^6 + \ldots$$

where C is the curvature (inverse of radius) of the base sphere at the optical axis, and $A_4$, $A_6$, ... are 4th, 6th, etc order aspheric terms. The first term in the above equation describes the first order deviation of surface 138 from a sphere, while the other terms represent higher order corrections.

In various exemplary embodiments of the invention aspheric profile 138 is characterized by a conic constant in a range of from about -1.1 to about -3, more preferably from about -1.1 to about -1.37, inclusive. Typical values for C are, without limitation, from about 0.006 $mm^{-1}$ to about 0.1 $mm^{-1}$ (in absolute value). The values for the higher order

aspheric terms can be selected such that the contribution of higher order term is less than 10% from the value of Z(s).

The term "toric profile" is also well known to those skilled in the art. To the extent that any further explanation may be required, this term is employed herein to refer to a radial profile of a surface having a first refracting power along a first meridian and a second refracting power along a second meridian, wherein the first and second meridians are perpendicular to each other and wherein the first and second refracting power differ from each other. Typically, the shape of surface 104, 140 is approximately that of a lateral section of a torus.

From a mathematical standpoint, a toric profile can be characterized by two conic constants $k_1$ and $k_2$, each corresponding to one of the two meridians.

For given conic sections $k_1$ and $k_2$, the sag *toric*(r, θ) of surface 104, 140 at any point (r, θ), r being the radial distance from the optical axis of lens device 100 and θ being the angle measured from the main meridian, can be written as:

$$toric(r,\theta)=\frac{(c_1\cos^2\theta+c_2\sin^2\theta)r^2}{1+\sqrt{1-(1+k_1){c_1}^2r^2\cos^2\theta-(1+k_2){c_2}^2r^2\sin^2\theta}}$$

where $c_1$ and $c_2$ are the curvatures along the respective meridians.

Typical values for $c_1$ and $c_2$ are, without limitation, from about 0.006 $mm^{-1}$ to about 0.1 $mm^{-1}$ (in absolute value). Typical values for $k_1$ and $k_2$ are, without limitation, from about -1.1 to about -3, or from about -1.1 to about -1.37, inclusive.

In some embodiments of the invention first side 138 has a diffractive pattern 144 formed thereon. Optionally and preferably, diffractive pattern 144 covers at least 80% or at least 90% or at least 95% or at least 99%, *e.g.,* the entire area of surface 102. FIG. 4 is a schematic illustration of an embodiment of the present invention in which diffractive pattern 144 covers a part of surface 138.

Diffractive pattern 144 is preferably selected so as to provide far focus power X where X is any power from +0D to about +35D. In various exemplary embodiments of the invention diffractive pattern 144 is selected to provide add-power Y, where Y is any power from about +2D to about +4D.

The term "add-power" is well known to those skilled in the art of multifocal lenses. To the extent that any further explanation may be required, add-power refers to the amount of optical power difference between the far focus power and the near-focus power.

Preferably, X and/or Y are integers or half-integers (e.g., X = 0D, 0.5D, 1D, ..., and/or Y=2D, 2.5D, 3D, ...).

In some embodiments of the present invention the toric profile 140, 102 is devoid of spherical aberration, wherein a cylinder value characterizing lens body 100 is at least 1 diopter or at least 2 diopters or at least 2.5 diopters or at least 3 diopters or at least 3.5 diopters or at least 4 diopters or at least 4.5 diopters or at least 5 diopters or at least 5.5 diopters or at least 6 diopters or any customized power to compensate corneal astigmatism.

A cylinder value of a lens measures the deviation from sphericity of a particular part of the lens's surface. The term "cylinder" originates from cylindrical lenses which inherently have different focal lengths in different directions. The optical effect caused by a lens having a non-zero cylinder value is known as surface astigmatism. In optometry nomenclature, the term "astigmatism" is also used to describe a physiological defect, for example, when the cornea has an irregular curvature. For subjects with astigmatism, a certain cylinder value in the lens is desired since it corrects the eye's astigmatism.

A toric surface which is devoid of spherical aberration is also known in the literature as an "aberration neutral" surface. Use of aberration neutral surface is unlike conventional IOL devices which employ negative asphericity so as to compensate for the shape of the cornea. A judicious selection of the conical constants $k_1$ and $k_2$ allows the use of an aberration neutral surface at the toric side, while maintaining adequate cylinder value.

The lens device of the present embodiments can be fabricated in any technique known in the art. Generally, the diffractive lens 144 may be constructed on a substance using a plurality of concentric annular zones separated by slanted steps, wherein the concentric zones effect both diffraction and refraction of incident light, while the steps are substantially devoid of any diffractive or refractive power. The substance on which the zones and steps are formed can be an unprocessed or partially processed lens body, in which case the formation of zones and steps serves for forming the lens device directly. Alternatively, the substance can be a mold, in which case the formation of zones and steps serves for forming a lens mold for mass fabrication of lens devices. In these embodiments, the lens device can be cased using the lens mold, as known in the art.

The formation of zones and steps may be done by any convenient manufacturing means, including, for example, a computer-controllable manufacturing device, molding or the like.

A "computer controllable manufacturing device" refers to a device that can be controlled by a computer system and that is capable of producing directly a lens body or a mold for producing a lens device. Any known, suitable computer controllable manufacturing device can be used in the invention. Exemplary computer controllable manufacturing devices

includes, but are not limited to, lathes, grinding and milling machines, molding equipment, and lasers. In various exemplary embodiments of the invention a Computerized Numeric Controlled (CNC) lathe machine is used, such as the lathers marketed under the trade names DAC™ Vision, Optoform and CareTec.

A Fast Tool Servo (FST) is optionally employed to form the toric profile. In these embodiments, the lathe machine can generate the diffractive pattern and the aspheric profile, and the FTS can generate the toric profile.

In general, ophthalmic and ocular devices differ from spectacles (eye-glasses) and other optical devices, by being designed to be in direct contact with the living tissue of the eye or its immediate surroundings. The devices which come in such direct contact are required to be biocompatible, as discussed herein, as well as to have certain physical properties with respect to their mechanical reshapability and reformability, flexibility, water-absorption capacity, and the likes. Implantable devices differ from non-implantable devices mainly by their mode of use and administration as well as their term of use, namely the devices that require a surgical procedure in order to be put in place are typically referred to as implantable. Implantable ophthalmic and ocular devices are expected to last longer and thus the requirements for their stability and compatibility in all aspects are much higher.

In principle, the multifocal ophthalmic device can be prepared from any polymeric or co-polymeric composition which is formulated to confer the required and desired chemical and mechanical attributes, as discussed herein.

A composition used in ophthalmic applications is typically formulated to be bio-compatible, chemo-compatible and physico-compatible so as to be adequate for use within the above-described ophthalmic device, or any other ophthalmic and ocular devices.

The term "bio-compatible", as used in reference to polymeric or co-polymeric compositions as presented herein, refers to the non-toxic and benign effect that the composition has on a living tissue (e.g., an eye or a portion or a component thereof) when in contact therewith.

The term "chemo-compatible", as used in reference to polymeric or co-polymeric compositions as presented herein, refers to the long-term non-leachability of unreacted components and diluents, non-degradability and overall long-term chemical stability of the composition when in contact with a living tissue in the eye and exposed to ambient light.

The term "physico-compatible", as used in reference to polymeric or co-polymeric compositions as presented herein, refers to the optical properties of the compositions in terms of refractive index, internal reflections, light transmission and/or absorbance, and other properties relating to light-matter interaction.

The present inventors have contemplated a formation of a multifocal device as described herein from polymeric and co-polymeric compositions designed suitable for use in such a device.

The polymeric or co-polymeric compositions presented herein, are typically derived from a mixture of monomers. In polymeric compositions, the monomers in the mixture are the same whereby in co-polymeric compositions several different classes of monomers are used As in any polymer, the polymeric or co-polymeric composition is based on plurality of polymeric backbones, each of which consists of a plurality of backbone units which are covalently attached to one another, and each of the backbone units comprises a plurality of building-blocks or polymerized monomeric units. Hence, the a polymeric or co-polymeric composition as presented herein, which is the outcome of polymerizing a pre-polymerization mixture of monomers, can be characterized and defined at the chemical and physical property level, in terms of the pre-polymeric mixture, namely its constituents prior to polymerization.

The monomers which are put into the pre-polymerization mixture are still unreacted (not yet polymerized) and are regarded as the starting materials for the compositions presented herein. In the case of a co-polymeric composition as presented herein, each of the monomers, identified and classified into classes, can be characterized by chemical and physical properties exhibited by the corresponding homopolymer which is derived from each of the individual monomer in the class. Hence, the co-polymeric compositions presented herein can be characterized by the monomer class which it is derived from, as well as the relative amount-ratios between each monomer class in the pre-polymerization mixture.

As used herein, the term "monomer" is used to describe a constituent of the pre-polymerization mixture which affords, upon polymerization, the polymeric or co-polymeric compositions presented herein. It is noted herein that while some of the constituents of the pre-polymerization mixture participate in the polymerization process as reactants which form covalent bonds with other constituents in the mixture during the polymerization process, some may not react with other constituents in a covalent-bond-forming reaction, but become embedded or incorporated within the matrix of the polymeric or co-polymeric composition, as will be discussed hereinbelow.

According to some embodiments of the present invention, a pre-polymerization mixture as described herein may include any of the monomers as described herein, as a starting material, in its monomeric form, or, alternatively, as a polymeric building block, which typically has a relatively low average molecular weight of about less than 2000 Daltons (as measured via gel permeation chromatography refractive index detection). According to other embodiments of the present invention, the starting material can include dimmers of a monomer (two monomeric units or building blocks) and in some cases be a short oligomer of building blocks, including oligomers made from more than one type of monomeric unit. Short oligomers are commonly referred to in the art as "blocks".

Some of the above-described embodiments therefore relate to a multifocal ophthalmic device which includes

a lens body which is formed with a plurality of concentric annular zones separated by slanted steps, wherein the concentric zones effect both diffraction and refraction of incident light, while the steps are substantially devoid of any diffractive or refractive power, as described herein, and wherein the lens body is made of a polymeric or co-polymeric composition which includes a polymeric backbone composed of a plurality of backbone units covalently linked to one another, which is derived from a pre-polymerization mixture of monomers having an improved formulations as presented hereinbelow. Optionally, according to some embodiments, the polymeric or co-polymeric composition includes at least one curcuminoid compound, as presented hereinbelow, incorporated in the composition or on the lens body as a mean to provide UV-light stabilization.

Hydrophobic Co-polymeric Composition

According to some embodiments of the present invention, the lens body of the multifocal ophthalmic device presented herein is made of a co-polymeric composition which is uniquely designed suitable for ophthalmic devices. As discussed hereinabove, these co-polymeric compositions presented herein are formulated to be bio-compatible, chemo-compatible and physico-compatible.

Hence, according to an aspect of some embodiments of the present invention, there is provided a multifocal ophthalmic device, as described herein, wherein the lens body of the device comprises a co-polymeric composition which is suitable for use in ophthalmic applications.

In some embodiments, the co-polymeric composition is based on five matrix-forming constituents (monomers) as well as other ingredients such as catalyst, UV-stabilizer/blocker and high energy visible blue light stabilizer. The compositions may further comprise colorant/dye additives, leachable agents (drugs) and the likes.

It is noted that the co-polymeric composition of the present embodiments is compatible for use in the eyes and is optically clear and suitable for use as material of construction of ophthalmic and ocular device. By optically clear it is meant that the composition is essentially transparent to visible light, as described and defined herein.

The co-polymeric composition presented herein typically and advantageously exhibits relatively high refractive indexes when in its final form and fully hydrated. The refractive index of the final and fully hydrated co-polymeric composition presented herein, is typically greater than about 1.5, more typically greater than about 1.51, still more typically greater than about 1.52 and even possibly greater than 1.53 or even 1.54, wherein the refractive index of the fully hydrated composition is measured at 25 °C in accordance with ASTM D 542 - 00 (2006).

Hence, according some embodiments of the present invention, the lens body of the multifocal ophthalmic device presented herein comprises a co-polymeric composition, the composition being derived from a mixture of monomers that includes:

a first aromatic acrylate (aryl acrylate) monomer characterized as forming, upon polymerization thereof, a first homopolymer which exhibits a refractive index that ranges from 1.50 to 1.53;
a second aromatic acrylate (aryl acrylate) monomer characterized as forming, upon polymerization thereof, a second homopolymer, which exhibits a Tg (glass transition temperature) lower than the Tg of the homopolymer derived from the first monomer by a range of 2 to 30 degrees centigrade;
a third monomer characterized as forming, upon polymerization thereof, a third homopolymer, which exhibit a Tg lower than 35 °C or lower than 37 °C;
a fourth monomer characterized as forming, upon polymerization thereof, a fourth homopolymer, which exhibit a capacity to absorb water to at least 20 % of its dry weight; and
a fifth monomer serving a crosslinking agent.

According to some embodiments of the invention, a concentration of the first aromatic acrylate monomer ranges from 50 % to 60 % of the total weight of the composition;

a concentration of the second aromatic acrylate monomer ranges from 15 % to 20 % of the total weight of the composition;
a concentration of the third monomer ranges from 10 % to 15 % of the total weight of the composition;
a concentration of the fourth monomer ranges from 5 % to 10 % of the total weight of the composition;
a concentration of the fifth monomer ranges from 2 % to 5 % of the total weight of the composition.

It is noted that the co-polymeric compositions presented herein, which are formed from a number of monomer classes, referred to herein as a first, second, third, fourth and fifth monomers, can include a variety of different monomers of each class, namely one or any number of monomers mentioned within the class of monomers corresponding to the first, second, third, fourth or fifth monomer.

Unless otherwise stated, the percentages (e.g., weight percentages) of the constituents of the co-polymeric

composition presented herein are denoted as weight percentages of the starting material with respect to the total weight of the pre-polymerizable mixture.

According to some embodiments of the present invention, the co-polymeric composition presented herein contains less than 75 % in total of aryl acrylate monomers, keeping the major component of the co-polymeric composition based on aryl acrylate, as opposed to a mixture of aryl methacrylate and aryl acrylate known in the art, which is less suitable for ophthalmic applications in general and for the multifocal ophthalmic device presented herein.

One disadvantage of using aryl acrylate and aryl methacrylate/acrylate in more than 80 % concentration is the occurrence of internal reflections and glare which may appear in a multifocal ophthalmic device made from such co-polymeric composition. This disadvantage is mitigated by controlling the content of the aryl (aromatic) monomer in the composition.

In general, every monomer adds from its associated property to co-polymeric composition, and when all property adds up in a prerequisite proportion, they form a co-polymer suiting the application.

The fifth monomer (class) is a crosslinking agent, as defined hereinbelow, interchangeably referred to herein as a crosslinker, which is characterized according to its capacity to alter the strength, rigidity and consistency of the co-polymeric composition presented herein. Thus, the crosslinking monomer (the fifth monomer) is a component having an effect on controlling flexibility of the obtained material for a soft embodiment of composition presented herein, giving the desired mechanical strength, improving capability of deformation recovery, and increasing co-polymerizable property with components for polymerization. Co-polymeric compositions which are not cross-linked may deteriorate rapidly as polymer chains are loosely held and increasing the possibility of getting extracted out under soxhlation extraction (gel content), resulting into loss of strength, loss of shape recovery and higher occurrence of vacuoles. The crosslinker is also the constituent that leads to an increase in the molecular weight of the composition (size of an average contiguous chain) by tethering chains to one-another. The molecular weight of the composition has a direct effect on the glistening, mechanical properties, refractive index and many other mechanical and optical properties of the composition and subsequently a device made therefrom.

The first monomer (class) is an aromatic (aryl-containing) acrylate type monomer (hence, a first aromatic monomer) which is characterized as forming, upon polymerization thereof, a first homopolymer having a refractive index between 1.50-1.53 (a criterion for selecting the first monomer). As a constituent of the co-polymeric compositions presented herein, the first monomer can include one or more monomer structures, namely different monomers wherein each satisfies at least the aforementioned criterion.

Acrylate monomers constitute a family which is a type of vinyl monomers, or esters which contain a vinyl group, namely two carbon atoms double-bonded to each other, directly attached to the carbonyl carbon of a carboxyl group, as illustrated in Scheme 1 below.

Scheme 1

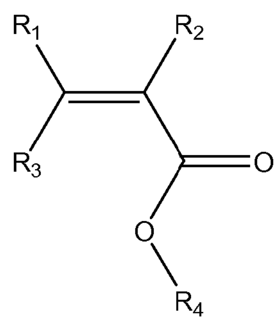

The term "methacrylate" refers to an acrylate monomer having a methyl group at position R2 in Scheme 1 above.

The phrase "aromatic acrylate", as used herein, refers to an acrylate ester having an aromatic substituent attached to the carbonyl, denoted R4 in Scheme 1 above.

Accordingly, the phrase "aromatic methacrylate" refers to a monomer as illustrated in Scheme 1 above, wherein R2 is a methyl group and R4 is an aryl or a heteroaryl group.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl

group may be substituted or unsubstituted.

Without being bound by a particular theory, the rationale behind using aryl ether acrylate monomers is their relatively flexible result-polymer compared to straight chain aryl alkyl methacrylate which adds to greater deforming ability into lens matrix without significantly compromising on refractive index and hydrophobicity.

Exemplary monomers that are suitable for use as a first monomer according to embodiments of the invention include 2-phenoxyethyl acrylate, 2-phenoxy ethyl methacrylate, 2-benzyloxy ethyl acrylate, 2-benzyloxy ethyl methacrylate and combinations thereof.

Other non-limiting examples of the first aromatic acrylate monomer according to some embodiments of the present invention, include 2-ethylphenoxy methacrylate; 2-ethylphenoxy acrylate; 2-ethylthiophenyl methacrylate; 2-ethylthiophenyl acrylate; 2-ethylaminophenyl methacrylate; 2-ethylaminophenyl acrylate; phenyl methacrylate; phenyl acrylate; benzyl methacrylate; benzyl acrylate; 2-phenylethyl methacrylate; 2-phenylethyl acrylate; 3-phenylpropyl methacrylate; 3-phenylpropyl acrylate; 4-phenylbutyl methacrylate; 4-phenylbutyl acrylate; 4-methylphenyl methacrylate; 4-methylphenyl acrylate; 4-methylbenzyl methacrylate; 4-methylbenzyl acrylate; 2-2-methylphenylethyl methacrylate; 2-2-methylphenylethyl acrylate; 2-3-methylphenylethyl methacrylate; 2-3-methylphenylethyl acrylate; 24-methylphenylethyl methacrylate; 2-4-methylphenylethyl acrylate; 2-(4-propylphenyl)ethyl methacrylate; 2-(4-propylphenyl)ethyl acrylate; 2-(4-(1-methylethyl)phenyl)ethyl methacrylate; 2-(4-(1-methylethyl)phenyl)ethyl acrylate; 2-(4-methoxyphenyl)ethyl methacrylate; 2-(4-methoxyphenyl)ethyl acrylate; 2-(4-cyclohexylphenyl)ethyl methacrylate; 2-(4-cyclohexylphenyl)ethyl acrylate; 2-(2-chlorophenyl)ethyl methacrylate; 2-(2-chlorophenyl)ethyl acrylate; 2-(3-chlorophenyl)ethyl methacrylate; 2-(3-chlorophenyl)ethyl acrylate; 2-(4-chlorophenyl)ethyl methacrylate; 2-(4-chlorophenyl)ethyl acrylate; 2-(4-bromophenyl)ethyl methacrylate; 2-(4-bromophenyl)ethyl acrylate; 2-(3-phenylphenyl)ethyl methacrylate; 2-(3-phenylphenyl)ethyl acrylate; 2-(4-phenylphenyl)ethyl methacrylate; 2-(4-phenylphenyl)ethyl acrylate; 2-(4-benzylphenyl)ethyl methacrylate; and 2-(4-benzylphenyl)ethyl acrylate, and the like.

Additional examples for suitable first monomers include naphthyl acrylates, dicyclopentyloxy acrylates, dicyclopentyl acrylates, nonylphenoxy polyethyleneglycol 200 acrylates, nonylphenoxy polyethyleneglycol 400 acrylates, alkoxylated phenol acrylates, 2-methacryloyloxyethyl 2-hydroxy propyl phthalates, 2-acryloxy ethyl-2-hydroxy ethyl phthalates, 2-hydroxy-3-phenoxy propyl acrylates, neopentyl glycol benzoate acrylates and the likes.

According to some embodiments of the present invention, the concentration of the first monomer ranges from 52 % to 59 % of the total weight of the composition.

The second monomer (class) is another aromatic acrylate monomer characterized as forming, upon polymerization thereof, a second homopolymer having a Tg lower than the Tg of the first homopolymer, which is derived from the first monomer, by 2 to 30 degrees centigrade (°C).

Non-crystalline polymeric solids are referred to as amorphous materials (atoms or molecules are not arranged in a lattice that repeats periodically in space). For all amorphous solids, whether glasses, organic polymers, and even metals (although having a lattice), Tg is the critical temperature that separates their glassy and rubbery behaviors. A glass is defined as a material that has no long-range atomic or molecular order and is below the temperature at which a rearrangement of its atoms or molecules can occur. On the other hand, a rubber is a non-crystalline solid whose atoms or molecules can undergo rearrangement. If a material is at a temperature below its Tg, large-scale molecular motion is not possible because the material is essentially frozen. If it is at a temperature above its Tg, molecular motion on the scale of its repeat unit (such as 50-mer in a polymer) takes place, allowing it to be "soft" or "rubbery". It is noted that the term "Tg" is applies herein to non-crystalline solids, which are mostly either "glasses" or "rubbers".

Hence, the phrases "glass transition temperature" or "rubber-glass transition temperature", as used herein in the context of polymers, refers to the temperature at which the Gibbs free energy is such that the activation energy for the cooperative movement of about 50 elements (50-mer) of the polymer is exceeded compared to a reference point, meaning that molecular chains are able to slide past each other when a force is applied. A glass transition temperature of a non-crystalline material, such as a polymer, is the critical temperature at which the material changes its behavior from being "glassy" to being "rubbery", while lowering the temperature across the Tg affords vitrification. "Glassy" in this context means hard and brittle (and therefore relatively easy to break), while "rubbery" means elastic and flexible and can absorb kinetic energy without shuttering.

According to some embodiments of the present invention, the chemical structure of the second monomer can follow the same chemical rational characterizing the first monomer, with the difference that the second monomer is selected according to the Tg characterizing the second homopolymer being lower than the Tg of the first homopolymer by 2-30 °C.

The choice of a second aromatic monomer according to embodiments of the invention include, depends on the choice of the first monomer as difference in relative Tg is the criterion for selecting the second monomer. Therefore, there is an overlap in the range of options for the first and the second monomers.

Exemplary monomers that are suitable for use as a second monomer according to embodiments of the invention include, but are not limited to 2-phenylethyl acrylate, benzyl acrylate, 2-chlorophenyl acrylate, 4-methyl benzyl acrylate, 2,4,6-tribromophenyl acrylate, pentabromophenyl acrylate and any combinations thereof.

According to some embodiments of the present invention, the concentration of the second monomer ranges from 15 % to 19 % of the total weight of the composition.

It is noted herein that some ophthalmic and ocular devices, and particularly implantable devices, are required to avoid posterior capsular opacification (PCO) after cataract replacement surgery. This adverse effect relates also to the tackiness of the composition. To control tackiness, suitable monomers are selected for the co-polymeric composition, while not compromising the refractive index of the resulting composition. Hence, the co-polymeric composition presented herein exhibits tackiness to some degree so as to avoid PCO, and therefore tackiness should be high enough to reduce PCO, and low enough so as not to hinder handling.

Thus, the first monomer is meant to be restricted to the above-mentioned range, since the first monomer is characterized by a relatively higher Tg which makes the resulting polymer less tacky than the polymer resulting from the second monomer. These formulation restrictions confer relatively low tackiness without compromising the target refractive index. At the same time, the reason for keeping a high Tg monomer (higher than a low Tg monomer), is to confer mechanical strength to the multifocal ophthalmic device, which increases due to higher crystallinity imparted to the resulting polymer.

It is further noted herein that according to some embodiments of the present invention, a relatively high Tg aryl monomer is used as a major constituent, while the prior art teaches the use of lower Tg aryl monomer as a major part of the composition. For example, U.S. Patent No. 5,290,892 teaches the use of 2-phenyl ethyl acrylate in higher amount compared to 2-phenyl ethyl methacrylate, while methacrylate compounds have higher Tg compared to corresponding acrylate compound. Together it forms at least 80 % of the composition taught in U.S. Patent No. 5,290,892.

The third monomer (class) is characterized as forming, upon polymerization thereof, a third homopolymer having a Tg lower than 37 °C. Thus, the third monomer is the component which has a notable effect on the flexibility of the obtained composition of the multifocal ophthalmic device presented herein, conferring softness and improving its capability to recover from deformation. The capacity to recover quickly from deformation (reformability) is required by an ophthalmic device when applied to the eye after folding and while following the shape shifts of the eye.

Exemplary monomers that are suitable for use as a third monomer according to embodiments of the invention include, but are not limited to, cellosolve methacrylate, methoxy ethyl acrylate, polyethylene glycol monomethacrylate, 1-dihydroxyperflurobutyl methacrylate, 2,5-dibromopropyl methacrylate, hexyl methacrylate, glycerol monomethacrylate, trifluroethyl methacrylate, butyl methacrylate, n-ocyl/isooctyl methacrylate, n-decyl/isodecyl methacrylate, ethyl methacrylate, ethylene triglycol methacrylate, butyl diglycol methacrylate, methoxy polyethylene glycol 350 methhacrylate, methoxy polyethylene glycol 500 methhacrylate, methoxy polyethylene glycol 1000 methhacrylate, methoxy polyethylene glycol 2000 methhacrylate. methoxy polyethylene glycol 5000 methhacrylate, polypropylene glycon methacrylate, ethoxytriglycol methacrylate, 2-ethoxyethoxy ethyl methacrylate, methoxy triethyleglycol methacrylate, phenoxy polyethylene glycol monomethacrylate and any combinations thereof.

According to some embodiments of the present invention, the concentration of the third monomer ranges from 11 % to 15 % of the total weight of the composition.

It is noted herein that use of aryl ether acrylate and aryl alkyl acrylate, such as 2-phenoxy ethyl acrylate and 2-phenyl ethyl acrylate, gives superior results over the use of methacrylate and acrylate of same pendant group as described in the art.

Methacrylate groups are known to increase the strength and rigidity of the resulting composition, since methacrylate compounds exhibit side chain crystallization thereby increasing strength and rigidity. According to some embodiments of the present invention, it is suggested herein to introduce high Tg aryl ether acrylate monomer which also imparts flexibility to the co-polymeric composition, enabling 20D IOL delivery even through a sub 2 mm incision in a wound assisted surgical technique. An additional advantage of using aryl ether acrylate is its relatively less tacky nature compared to aryl alkyl methacrylate.

By using lesser amount of aryl acrylate monomer, polymer of relatively low refractive index is prepared to avoid glare/internal reflection problem at the same time, refractive index is maintained to a level that it would enable the lens to go through sub 2 mm incision.

For increasing strength and reformability, monomers like methoxy ethyl methcaylate are used. Its Tg is lower than 37 °C and it forms relatively hydrophobic polymer than conventional 2-ethoxy ethyl methacrylate. In general, in the pendant ether group, odd number of methylene (CH2) gives optimal results. Examples include methoxy ethyl acrylate, propoxy ethyl acrylate, pentoxy ethyl acrylate and the likes.

The fourth monomer (class) is a hydrophilic monomer which is characterized as forming, upon polymerization thereof, a fourth homopolymer exhibiting a capacity to absorb water to at least 20 % of its dry weight.

Water content of the homopolymer made from hydrophilic monomer should absorb enough so as to conform to the requirement of ophthalmic devices such as contact lenses and IOLs. After extraction of the leachables from the multifocal ophthalmic device, a process that is discussed hereinbelow, vacuoles may form in the product. Hence, all hydrophobic polymeric compositions exhibit vacuoles and some water absorption. When ophthalmic device comes in a contact with water or another aqueous medium, these media would tend to concentrate at the vacuoles.

Prime purpose of using a hydrophilic monomer is to uniformly disperse the water in the matrix. After putting the ophthalmic device in physiological medium like normal saline or highly pure water, these vacuoles give rise to white spots. To overcome this problem, hydrophilic monomer would disperse water uniformly rather than allowing water to concentrate in voids and vacuoles. This uniform dispersion gives rise to clear and spotless lenses. It also helps to increase the strength of the matrix and to control its tackiness.

Some commonly available hydrophilic monomer absorbs more than 20 % of the total weight of their corresponding homopolymer. If the required water content in the multifocal ophthalmic device should be kept bellow 20 %, other monomers can be selected so as to counter-effect the absorption of water.

The forth monomer is also effective to reduce tackiness of the co-polymeric composition presented here, as well as to improve its mechanical properties, as well as to uniformly disperse water molecules throughout the matrix at a wide temperature change.

As used herein, the phrase "hydrophilic monomer" refers to compounds which produce hydrogel-forming homopolymers, namely homopolymers which become associated with substantial amounts of water (for example, at least 20 % based on the weight of the dry homopolymer), and which physically swell as a result of such association.

Exemplary fourth monomers include, without limitation, alkoxy alkyl (meth)acrylate; N-vinyl pyrrolidone; hydroxyalkyl acrylates and hydroxyalkyl methacrylates, such as 2-hydroxyethyl acrylate, 2-hydroxyethyl methacrylate, 3-hydroxypropyl acrylate, 3-hydroxypropyl methacrylate, 4-hydroxybutyl acrylate, 4-hydroxybutyl methacrylate, 2,3-dihydroxypropyl acrylate, 2,3-dihydroxypropyl methacrylate and the like; acrylamide; N-alkyl acrylamides such as N-methyl acrylamide, N-ethyl acrylamide, N-propyl acrylamide, N-butyl acrylamide and the like; acrylic acid; methacrylic acid; and the like and mixtures thereof.

Additional exemplary monomers that are suitable for use as a fourth monomer according to embodiments of the invention include, but are not limited to, hydroxyl ethyl methacrylate, glycerol monomethacrylate, ethylene triglycol methacrylate, butyl diglycol methacrylate, methoxy polyethylene glycol 350 methhacrylate, methoxy polyethylene glycol 500 methhacrylate, methoxy polyethylene glycol 1000 methhacrylate, methoxy polyethylene glycol 2000 methhacrylate, methoxy polyethylene glycol 5000 methhacrylate, polypropylene glycon methacrylate, ethoxytriglycol methacrylate, methoxy triethyleglycol methacrylate, phenoxy polyethylene glycol monomethacrylate and any combinations thereof.

According to some embodiments of the present invention, the concentration of the fourth monomer ranges from 7 % to 9 % of the total weight of the composition.

As used herein, the phrase "cross-linking monomer" refers to a substance that promotes or regulates intermolecular covalent, ionic, hydrophobic or other form of bonding between polymer chains, linking them together to create a network of chains which result in a more rigid structure. Crosslinking monomers, according to some embodiments of the present invention, contain at least two reactive groups that are reactive towards a variety of groups, including double bonds, sulfhydryls and amines, and create chemical bonds between two or more polymer molecules. Crosslinking monomers include homo-bifunctional crosslinking monomers that have two identical reactive end groups, and hetero-bifunctional crosslinking monomers which have two different reactive end groups. These two classes of crosslinking monomers differ primarily in the chemical reaction which is used to effect the crosslinking step, wherein homo-bifunctional crosslinking monomers will require a one step reaction, and hetero-bifunctional crosslinking monomers will require two steps to effect the same. While homo-bifunctional crosslinking monomers have the tendency to result in self-conjugation, polymerization, and intracellular cross-linking, hetero-bifunctional agents allow more controlled two step reactions, which minimize undesirable intramolecular cross reaction and polymerization. Crosslinking monomers are further characterized by different spacer arm lengths. A crosslinking monomer with a longer spacer arm may be used where two target groups are further apart and when more flexibility is desired.

Exemplary crosslinking monomers include, without limitation, butanediol di(meth)acrylate, ethylene glycol di(meth)acrylate, diethylene glycol di(meth)acrylate, triethylene glycol di(meth)acrylate, propylene glycol di(meth)acrylate, dipropylene glycol di(meth)acrylate, diallyl fumarate, allyl (meth)acrylate, vinyl (meth)acrylate, trimethylolpropane tri(meth)acrylate, methacryloyloxyethyl (meth)acrylate, divinylbenzene, diallyl phthalate, diallyl adipate, triallyl diisocyanate, $\alpha$-methylene-N-vinylpyrrolidone, 4-vinylbenzyl(meth)acrylate, 3-vinylbenzyl (meth)acrylate, 2,2-bis((meth)acryloyloxyphenyl)hexafluoropropane, 2,2-bis((meth)acryloyloxyphenyl)propane, 1,4-bis(2-(meth) acryloyloxyhexafluoroisopropyl)benzene, 1,3-bis(2-(meth)acryloyloxyhexafluoroisopropyl)benzene, 1,2-bis(2-(meth) acryloyloxyhexafluoroisopropyl)benzene, 1,4-bis(2-(meth) acryloyloxyisopropyl)benzene, 1,3-bis(2-(meth) acryloyloxyisopropyl) benzene, 1,2-bis(2-(meth) acryloyloxyisopropyl)benzene, and the like. These crosslinking monomers can be used solely or in a combination use of two or more thereof. Among those, ethylene glycol dimethacrylate and butanediol diacrylate are widely use effect controllable flexibility, desired mechanical strength, improved capability of deformation recovery, and increased co-polymerizable property.

Additional exemplary monomers that are suitable for use as a fifth monomer according to embodiments of the invention include, but are not limited to ethylene glycol dimethacrylate, 1,4-butane diol diacrylate, glycerol dimethacrylate, allyl methacrylate, 1,6 heaxane diol diacrylate, 1,4-butanediol dimethacrylate, 1,6-hexane diol dimethacrylate and any combinations thereof.

According to some embodiments of the present invention, the concentration of the fifth monomer ranges from 2 % to 3.5 % of the total weight of the composition.

The amount of the fifth monomer (crosslinker) depends on selection of the prior monomers. It is added to have an optimal shape recovery, to lessen the extractables and reduce glistening. An excess amount may increase glistening of the multifocal ophthalmic device after introduction into physiological medium. Lesser amount may result into loss of strength, higher extractables (more vacuoles) and slower shape recovery.

In addition, the co-polymeric compositions presented herein can include a variety of additional or alternative ingredients, feature additives and the likes. Examples include, without limitation, UV blockers, dyes, light-stabilizers, coating materials, pharmaceuticals (therapeutic agents), cell receptor functional groups, protein groups, viscosity agents (e.g., thickeners or thinners), diluents, combinations thereof or the like.

As discussed hereinabove, each and every constituent in the co-polymeric compositions presented herein, as well as their relative proportion in the making thereof, contribute to the wide range of requirements and necessary characteristics of lens body of the device made from these compositions.

For example, according to some embodiments of the present invention, the lens body of the device made from the compositions presented herein exhibit visible light transmission of at least 97 % of incident visible light, as determined according to ASTM D 1003 standard and/or ISO 11979-2:2000 standard.

In addition, or alternatively, the lens body of the device made from the compositions presented herein exhibit a refractive index of at least 1.53, as determined according to the ASTM D 542-00(2006) standard.

In addition, or alternatively, according to some embodiments of the present invention, the compositions used to manufacture the lens body of the device presented herein exhibit a loop pull force mechanical strength of at least 60 grams, at least 50 grams or at least 40 grams, as determined according to the ISO 11979-3:2006 standard.

In addition, or alternatively, the compositions used to manufacture the lens body of the device presented herein are characterized by a glass transition temperature not higher than 5 °C, not higher than 10 °C or not higher than 15 °C, as determined according to the ASTM D3418-03:2000 standard.

In addition, or alternatively, Shore A hardness exhibited by lens body of the device prepared from the compositions according to some embodiments of the present invention, ranges from 77 to 80, as determined according to the ASTM D2240:2000 standard.

In addition, or alternatively, according to some embodiments of the present invention, the co-polymeric compositions presented herein exhibit an unfolding time of less than 6 seconds for full recovery of original shape injected through a sub 2 mm cartridge at room temperature. Such requirement is important when the device is placed in position by inserting it under leaving tissue with a narrow gauge cartridge (sub 2 mm).

When using the co-polymeric compositions according to some embodiments of the present invention, the resulting multifocal ophthalmic device is characterized by having essentially no internal reflections, and further have essentially no vacuoles and/or perceivable glistening as determined by visual inspection at a magnification of 50X.

One of the requirements of the multifocal ophthalmic device presented herein, involving leachable content, is clearly met by the co-polymeric compositions presented herein, which have a leachable content of less than 0.6 %, as determined according to the ISO 11979-5:2006 and/or the ISO 11979-5:2006 standards.

According to some embodiments of the present invention, the multifocal ophthalmic device made from the compositions presented herein are essentially tack-free, as determined according to the ASTM D 3654 standard.

The multifocal ophthalmic device made from the co-polymeric compositions presented herein can also be referred to as a hydrophobic diffractive ophthalmic device and can be, for example, a hydrophobic diffractive IOL.

According to some embodiments of the present invention, the co-polymeric composition for making the device or body lens of the device described herein further includes a radiation-resistant compound, which is typically referred to as a UV-blocker agent or additive, UV-light stabilizer and/or UV-absorbent agent. The terms "UV-blocker" and "UV-stabilizer", and grammatical diversions and inflections thereof, are used herein interchangeably, since stabilizing a polymeric composition against the degradation caused by UV-light stems also from the capacity to block UV-light by the composition.

The role of protection from UV damages, both to the eye (protect light sensitive retina) and the co-polymeric composition itself, can be taken by one type of UV-blocker or a combination of several different compounds, some embedded and some copolymerized with the composition. The UV-blocker can thus be a polymerizable constituent, which presents advantages in terms of leachability of the agent, and it can be an embedded constituent, namely incorporated consistently in the matrix of the polymeric composition so as not to leach out.

It is noted herein that UV-protection is particularly desirable for ophthalmic devices such as the multifocal ophthalmic device presented herein, which may be located within the eye for extended periods of time (e.g., greater than 6 months, a year, several years or more) as opposed to, for example, disposable contact lenses. As such, it is highly desirable for these types of devices to exhibit longer term resistance to degradation caused by radiation exposure.

An ultra-violet absorbing material (UV-blocker additive) can be any natural or synthetic compound which absorbs ultraviolet light, i.e., light having a wavelength shorter than about 400 nm, but does not absorb any substantial amount

of visible light. A natural UV-blocker can be a curcuminoid compound, as defined and discussed hereinbelow. The ultraviolet absorbing compound is incorporated into the monomer mixture and is embedded or entrapped in the polymer matrix when the monomer mixture is polymerized. According to some embodiments of the present invention, the UV-blocker provide a transmission cut-off above a wavelength of 385 and typically provide cut-off in the short wavelength visible (410-430 nm) region of the electromagnetic spectrum. Such chromophores can then provide desired protection to the human eye and/or the device material from UV radiation (<400 nm). Suitable UV-blockers can also be referred to as UV/short wavelength visible light absorbers, dye or chromophores.

Unless otherwise specified, "cut-off" means the wavelength at which light transmission does not exceed 1 %. "1 % cut-off' means the wavelength at which light transmission does not exceed 1 %. "10 % cut-off" means the wavelength at which light transmission does not exceed 10 %.

Typical ultraviolet absorbing compounds, based on synthetic chromophores, include substituted benzophenones, such as 2-hydroxybenzophenone, and 2-(2-hydroxyphenyl)benzotriazoles. According to some embodiments of the present invention, the ultraviolet absorbing compound may be co-polymerizable with the monomers and is thereby firmly embedded in the polymer matrix. In this way possible leaching of the ultraviolet absorbing compound out of the lens and into the interior of the eye is minimized. Suitable co-polymerizable ultraviolet absorbing compounds include substituted 2-hydroxybenzophenones as disclosed in, for example, U.S. Patent No. 4,304,895 (incorporated by reference as fully set forth herein) and 2-hydroxy-5-acryloxyphenyl-2H-benzotriazoles disclosed in U.S. Patent No. 4,528,311 Alternatively, the ultraviolet absorbing compound is 2-(3'-methallyl-2'-hydroxy-5'methyl phenyl) benzotriazole.

Other synthetic ultraviolet absorbing compounds include phenol-2-(5-chloro-2H-benozotriazol-2-yl)-6-(1,1-)dimethyl-4-methyl (Tinuvin® 326), 4-benzoyl-3-hydroxyphenyl-2-methacrylate, 2-[4-(2h-1,2,3-benzotriazol2-yl)-3-hydroxyphenoxy]ethyl-2-methacrylate and combination thereof.

Optic devices based on co-polymeric compositions may also comprise a polymerizable or embedded yellow dye that attenuates medium- to long-wavelength (430-500 nm) blue light. Such dyes and other useful chromophores are described in U.S. Patent No. 7,691,918, which is fully incorporated herein for all purposes.

Yellow dye gives a yellowish tint to lens; natural lens tends to get yellow as age of patient progresses. Yellow tinted artificial lens gives the elderly patient the appearance of a natural lens. The yellow dye also provides protection from visible blue light which may lead to age related macular degeneration.

Presently known UV-blocking additives and synthetic chromophores, used in polymeric compositions for ophthalmic and ocular devices, may suffer from one or more drawbacks, including biocompatibility, physical, mechanical and chemical stability, and manufacturing factors (e.g., cost and complex syntheses).

Polymeric and co-polymeric compositions containing Curcuminoid as a Natural UV-Blocker:

According some embodiments of the present invention, the lens body of the multifocal ophthalmic device presented herein, which comprises a co-polymeric composition (also referred to herein as a co-polymer or a hydrophobic co-polymeric composition) described herein, further includes a natural UV-blocker in the form of a curcuminoid compound incorporated in or on the co-polymeric composition and/or the lens body.

According to some embodiments of the present invention, the pre-polymerization mixture of monomers includes at least 50 percents acrylate monomers.

It is noted herein that the benefits of incorporating a curcuminoid compound in ophthalmic and ocular devices applies to any polymeric or co-polymeric composition or a lens body of a device comprising the same. Thus, an ophthalmic or ocular device, according to some embodiments of the present invention, can be made from any suitable polymeric or co-polymeric composition as known in the art, and the curcuminoid compound can be added to the pre-polymerized mixture of the composition before curing, or applied thereon after curing, as described herein.

According to another aspect of the present invention, there is provided a multifocal ophthalmic device which includes a lens body which is formed with a plurality of concentric annular zones separated by slanted steps, wherein the concentric zones effect both diffraction and refraction of incident light, while the steps are substantially devoid of any diffractive or refractive power, wherein the lens body is made of a polymeric or co-polymeric composition which includes at least one curcuminoid compound, as presented hereinabove, incorporated in the composition or on the lens body as a mean to provide UV-light stabilization.

The lens body of the multifocal ophthalmic device can be made from any suitable polymeric or co-polymeric composition as known in the art, and the curcuminoid compound can be added to the pre-polymerized mixture of the composition before curing, or applied thereon after curing, as described herein. The curcuminoid compound is therefore incorporated into the composition as described hereinabove.

In some embodiments, a polymeric or co-polymeric composition which includes a curcuminoid compound is derived from a pre-polymerization mixture of monomers that includes at least 50 weight percents acrylate monomers.

Exemplary acrylate monomers suitable for use in the context of these embodiments include, but are not limited to, an acrylate, a methacrylate, an aryl acrylate and an aryl methacrylate.

As demonstrated in the Examples section that follows, one exemplary co-polymer which includes a curcuminoid compound can be formed from one or more monomers such as, but not limited to, 2-phenoxy ethyl methacrylate (POEMA), cyclohexyl acrylate (CHMA) and 1,4-butane diol diacrylate (BDDA). An exemplary composition, according to some embodiments of the present invention, is formed, for example, from a pre-polymerization mixture containing 50-70 % 2-phenoxy ethyl methacrylate (POEMA), 20-50 % cyclohexyl acrylate (CHMA) and 1-5 % 1,4-butane diol diacrylate (BDDA), and 0.001-0.5 % curcuminoid compound, each measured by dry weight percentages of the total dry weight of the pre-polymerization mixture. Other constituents may also be included in minor amounts, such as a catalyst (a polymerization initiator agent), cross-linking monomers, colorant/dye additives and the likes.

Optionally, according to embodiments of this aspect, the co-polymeric composition includes a polymeric backbone composed of a plurality of backbone units covalently linked to one another, which is derived from a pre-polymerization mixture of monomers having a unique formulations as presented hereinabove.

Hence, according to another aspect of some embodiments of the present invention, there is provided a multifocal ophthalmic device which includes a lens body which is formed with a plurality of concentric annular zones separated by slanted steps, wherein the concentric zones effect both diffraction and refraction of incident light, while the steps are substantially devoid of any diffractive or refractive power, wherein the lens body is made of a hydrophobic co-polymeric composition, as described herein.

Combining the beneficial features imparted by the curcuminoid compound as described herein with the beneficial features of the co-polymeric composition described herein result in a vacuole free, glistening free, internal reflection free and tack free co-polymeric composition, which is protected from the damaging effects of UV light and meet the requirement of tensile strength, deformation recovery ability and mechanical, optical, biological and toxicological requirements.

As can be seen in the Examples section that follows, the present inventors have successfully incorporated curcumin in an exemplary co-polymeric composition which is suitable for use in ophthalmic and ocular devices such as the multifocal ophthalmic device discussed hereinabove, and were able to obtain a desirable level of transparency towards visible light and at the same time opacity with respect to ultraviolet light, using a relatively low concentration of curcumin.

Thus, it has been demonstrated that naturally occurring (natural) compounds such as curcuminoids, which are generally recognized as safe for human consumption and somatic use, can be used with certain polymers or co-polymers, such as substantially acrylate-based polymers, to form lasting compositions with suitable UV/blue light blocking and UV-light stabilization properties. It has been recognized that curcuminoids used in ophthalmic and ocular devices as blue/UV light blockers would overcome the limitations and possible impairments associated by the use of synthetic compounds such as diphenyl-azo-based, benzotriazole-based and benzophenone-based UV-blockers and the like.

It has been further demonstrated by some of the present inventors that curcuminoids can be used effectively even at low concentration in the polymeric composition, relative to the concentration required for benzophenone-based and benzotriazole-based UV-blockers, in order to achieve comparable effective UV-blocking. A low concentration of the UV-blockers not only affects the cost of the resulting product but also affects the visual clarity of the product and its final dimensions. Furthermore, the proven UV-blocking effectiveness of curcuminoids would make it highly suitable for in the multifocal ophthalmic device presented herein.

Thus, relatively low concentrations of the curcuminoids are sufficient to exhibit the desired activity. Hence, according to some embodiments of the present invention, the concentration of the curcuminoid compound in the composition ranges from 0.0002 weight percentage to 1 weight percentage or from 0.001 weight percentage to 0.5 weight percentage of the total weight of the composition. Any value lower than 1 weight percent is contemplated, hence any value lower than 0.9, 0.8, 0.7, 0.6, 0.5, 0.4, 0.3, 0.2, 0.1, 0.05, 0.001 and any value lower than 0,0005 weight percent is contemplated as well.

It has been further demonstrated that curcuminoids, unlike many known synthetic phenol-containing UV-blockers, do not inhibit or retard the rate and/or degree of polymerization, when added to the pre-polymerization mixture.

The use of compounds of the curcuminoid family allows selecting a compound with certain optical characteristics, such as, for example, a particular light-absorption range and a particular color or lack thereof.

Without being bound by any particular theory, it is suggested that the transparency of the co-polymeric composition is maintained upon incorporating curcumin therein due to the relatively low concentration of the embedded curcumin. Such low concentration is sufficient due to the effective UV-absorption characteristics of curcuminoids and the surprising finding that curcuminoids do not leach out of polymers or co-polymers derived from pre-polymerization mixtures having at least 50 percents acrylic monomers.

As further demonstrated in the Examples section that follows, curcumin was found to be incorporated firmly and consistently in an exemplary polymeric composition, as confirmed by the stability of its concentration in the tested composition before and after the tested composition was subjected to extended cycles of extraction in organic solvents which are known to dissolve curcumin.

The term "incorporated", as used herein, refers to the physical state of one substance in a composition containing other substances. In the context of some embodiments of the present invention, an incorporated curcuminoid compound

is incorporated within the co-polymer composition described herein such that the curcuminoid compound is at least partially surrounded by the co-polymeric composition and entrapped thereby.

In some embodiments, the incorporated curcuminoid compound is distributed within the polymeric or co-polymeric composition in a uniform and sustainable form, and is enclosed in the surrounding mass.

According to some embodiments of the present invention, the curcuminoid compound incorporated within the polymeric or co-polymeric composition is not covalently attached to one or more constituents of the polymeric or co-polymeric composition. In some embodiments, the curcuminoid compound interacts with the polymeric or co-polymeric composition via physical interactions, such as, for example, entanglement, absorption, adsorption and/or entrapment, and not via chemical interactions such as covalent, ionic, or hydrogen bonds.

Curcuminoids constitute a versatile group of chromophore-containing substances, which can be selected by their light absorption properties to suit a particular application.

In general, curcuminoids are polyphenols characterized by a pronounced yellow color, and most of the naturally occurring curcuminoids, including curcumin itself, have been recognized as generally safe for human consumption and suitable for pharmaceutical purposes.

The term "curcuminoid", as used herein, is used to collectively describe curcumin, as well as derivatized curcumin compounds. Derivatized curcumin compounds have a curcumin backbone structure, and optionally have one or more different chemical groups (substituents) attached at various positions of the curcumin backbone structure. A derivatized curcumin compound may differ from curcumin by chemical and physical characteristics, such as solubility, reactivity, light interaction and the likes, as a result of its substituents.

Curcuminoid compounds according to some embodiments of the present invention, can be collectively represented by General Formula I:

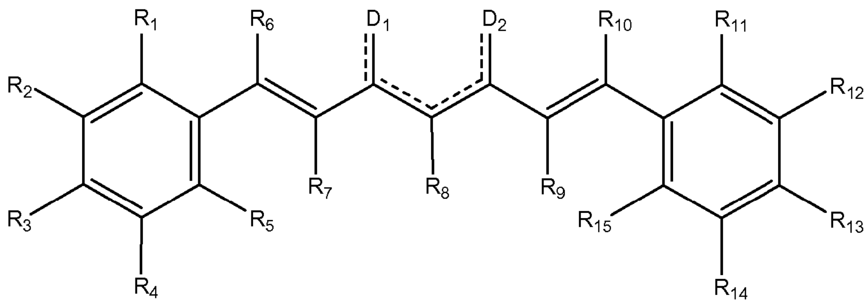

Formula I

According to some embodiments of the present invention, each of D1 and D2 is individually selected from the group consisting of O, N, S or C(aryl), whereas D1 and D2 may be connected directly or via a connecting atom to form a conjugated ring (aryl, heteroaryl etc.); and wherein each of R1-R15 is individually selected from the group consisting of alkyl, alkoxy and hydroxy.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted.

The curcuminoid compound utilized in embodiments of the present invention can be a naturally-occurring curcuminoid or a synthetically prepared curcuminoid, with naturally-occurring curcuminoids being more desirable.

Exemplary curcuminoid compounds that are suitable for use in the context of some embodiments of the present invention include, but are not limited to, curcumin (illustrated in the scheme below), bisdemethoxycurcumin, monodemethoxycurcumin and tetrahydroxycurcumin, which are all natural curcuminoids found in plants such as the curcuma species.

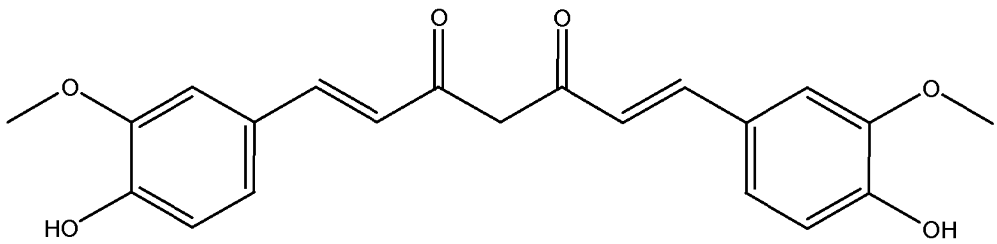

(1E,6E)-1,7-bis (4-hydroxy-3-methoxyphenyl)-6-heptadiene-3,5-dione

(Curcumin)

The naturally occurring curcuminoids differ from one another in both the number and position of the methoxy groups, and all exhibit a keto-enol tautomerism in the midsection of the chromophore chain. These curcuminoids differ in their light absorbing and free radical scavenging properties. All of the naturally-occurring curcuminoids may serve as efficient UV-blockers and/or stabilizers, in the context of embodiments of the present invention. For instance, some natural curcuminoid sources contain tetrahydroxycurcumin (THC), which is a colorless compound, which can be used as a UV stabilizer in applications where yellow color is not required or desired.

THC is a suitable curcuminoid compound, according to some embodiments of the present invention, in applications where yellowish tint is to be avoided.

Curcuminoid compounds can be extracted as naturally occurring substances or be prepared synthetically, and can be used as mixtures thereof or as isolated species. Curcuminoid compounds which are also encompassed by the present embodiments are disclosed in, for example, U.S. Patent No. 3,479,345, U.S. Patent Application Nos. 20030153512, 20060276536, 20070060644, 20070204412, 20080200478, 20100010232, 20100048901 20100048901 and 20100087527.

As discussed herein, substances that can leach (be extracted) out of a matrix may cause several adverse effects, such as harming the biological environment (tissue) surrounding the matrix, and reducing the effectiveness of the composition in, for example, blocking UV light. Hence, the characteristic behavior of the curcuminoid in polymeric or co-polymeric compositions can be referred to as low leachability (from the term "leachable") with respect to the matrix constituents and with respect to the curcuminoid compound, and can be defined in terms of comparison of concentrations before and after an extraction process.

Low leachability is significant when a substance is used in implantable devices, since in such devices, long-term performance is desired.

The process by which the incorporation of the curcuminoid compound is evaluated is also referred to as the extraction step of unreacted components and diluents (UCDs) from the cured composition, which is discussed in detail herein, and exemplified in the Example section that follows below.

According to some embodiments of the present invention, there is no perceivable difference in the concentration of the curcuminoid compound after the extraction process for removal of UCDs. Hence, according to some embodiments of the present invention, the polymeric or co-polymeric composition described herein as comprising a curcuminoid compound is such that the concentration of the curcuminoid compound in the composition does not decrease as a result of extraction step of UCDs, or decreases by no more than 0.0001 %, when subjected to an extraction process in an organic solvent, including also solvents which can readily dissolve the curcuminoid compound.

As discussed herein, a curcuminoid compound can be selected according to the desired light-absorption properties which are required from the composition.

According to some embodiments of the present invention, the curcuminoid can be selected such that the composition is substantially transparent to light at a wavelength ranging from about 400 to about 800 nm, and it can also be selected such that the composition is substantially opaque to light at a wavelength ranging from about 190 to about 440, or substantially opaque to light at a wavelength ranging from about 100 to about 400.

Such optical properties were demonstrated for compositions comprising curcumin, bisdemethoxycurcumin or monodemethoxycurcumin, as the curcuminoid compound.

According to some embodiments of the present invention, the curcuminoid can be selected such that the composition is substantially transparent to light at a wavelength ranging from about 490 to about 800 nm, and it can also be selected such that the composition is substantially opaque to light at a wavelength ranging from about 190 to about 440, or from about 100 to about 490. Such a composition is effective in reducing or essentially blocking the transmission of violet/blue light.

Such optical properties can be afforded when the curcuminoid compound is, for example, tetrahydroxycurcumin (THC).

As discussed above, UV-light is characterized by a wavelength ranging from 100-440 nm (some sources state that visible region starts from about 390 nm), violet is the color of the short-wavelength end of the human visible spectrum ranging approximately 380-450 nm, and blue light ranges 450-475 nm.

According to some embodiments of the present invention, the polymeric or co-polymeric composition as presented herein, which further comprises at least one curcuminoid compound as described herein is characterized by having UV-light blocking properties, wherein the UV-light is characterized by a wavelength ranging from 100 nm to 440 nm.

Some curcuminoid compounds exhibit yellow color which leads to absorption of visible blue and violet light. According to some embodiments of the present invention, the curcuminoid compound is such that it acts as a UV absorber as well as blue light blocker.

According to some embodiments, two different additives for radiation protection can be present in the composition, one for UV stabilization and another for visible blue light protection.

It is noted herein that the benefits of incorporating a curcuminoid compound in ophthalmic devices can apply also with body lens made from any polymeric or co-polymeric composition, as described herein.

The polymeric or co-polymeric composition for making lens body 12, according to some embodiments of the present invention, can be prepared by conventional polymerization techniques as known in the art, which include mixing the monomers and optional additives, such as UV-blockers, into a homogeneous pre-polymerization mixture, optional heating, degassing and adding additional ingredients, such a polymerization initiator such as a free radical polymerization initiator, and subjecting the mixture to polymerization conditions after casting the mixture into a mold.

These general processes allow adding any optional additive, such as the natural UV-blocker curcuminoid compound, at various steps of the process. For example, for preparing a co-polymeric composition as described herein, a mixture containing the five types of monomers is used. For preparing a polymeric or co-polymeric composition having a curcuminoid compound incorporated therein or thereon, a mixture containing aryl acrylate monomers as described herein is used.

Incorporation of the curcuminoid compound can be made while adding the curcuminoid compound to the pre-polymerization mixture or by contacting the composition with the curcuminoid compound and allowing it to be incorporated thereon.

Some processes of preparing the multifocal ophthalmic device further include post-polymerization steps such as chemical treatments, extractions and mechanical shaping.

However, some processes may be more suitable for forming multifocal ophthalmic devices made from particular compositions, as presented herein.

Hence, according to an aspect of embodiments of the present invention, there is provided a process of preparing the polymeric or co-polymeric composition presented herein, which is effected by:

admixing a pre-polymerization mixture containing the monomers presented hereinabove, as well as other optional constituents and additives and a free radical polymerization initiator;
optionally degassing the pre-polymerization mixture so as to remove any dissolved gasses which may interfere with optical clarity of the composition by forming vacuoles;
heating said pre-polymerization mixture while stirring;
optionally degassing the pre-polymerization mixture again so as remove volatile residues after heating;
optionally admixing an additional amount of the initiator into the pre-polymerization mixture so as to obtain a polymerization reaction mixture;
admixing a curing agent into the reaction mixture;
casting the reaction mixture into a mold;
exposing the reaction mixture in the mold to curing conditions, to thereby obtain the co-polymer composition presented herein; and
subjecting the co-polymeric composition to a multiple extraction so as to rid it from unreacted contaminants.

According to some embodiments of the present invention, the pre-polymerization mixture comprises the first, second, third, fourth, and fifth monomers in their appropriate ratios, as described herein for a hydrophobic co-polymeric composition.

According to some embodiments of the present invention, one of the additives can be a curcuminoid compound, as discussed hereinabove.

Optionally, heating the pre-polymerization mixture while stirring is performed at 40 °C until the viscosity of pre-polymerization mixture reaches an optimal level (120 cps at 25 °C). The viscosity measurements are obtained from the torque applied on the stirring device.

Once all the monomers and other components are mixed together for polymerization, the polymerization reaction may be initiated by adding a radical polymerization initiator in a conventional manner to obtain the polymeric or co-polymeric composition according to some embodiments of the present invention.

The choice of initiator also determines the kinetics of the polymerization reaction. As discussed hereinabove, the molecular weight of the composition imparts properties to the multifocal ophthalmic device, such as glistening, mechanical properties, refractive index and transmittance. Molecular weight is inversely proportional to the half power of the initiator concentration.

The initiator, also referred to herein as a catalyst, is typically employed to initiate the polymerization of the monomers and/or carry out the crosslinking or thermosetting of the polymeric or co-polymeric compositions formed of those monomers, as presented herein. Thus, according to some embodiments of the present invention, the co-polymeric composition present herein further includes an initiator (a catalyst).

According to some embodiments of the present invention, the polymerization reaction follows a free-radical propagation mechanism. As known in the art concerning conventional polymerization methods, the free-radical polymerization reaction may be initiated, for example, by free radical initiators, either thermally or photochemically. When using a thermally initiated free radical polymerization reaction, the method is typically effected by heating gradually from room temperature to an elevated temperature, such as 130 °C, and the temperature can be elevated stepwise and/or cycled. When the polymerization initiator is controlled photochemically, the polymerization reaction in initiated by irradiating the pre-polymerization mixture with electromagnetic radiation, such as microwave, ultraviolet light or radiation ($\gamma$ ray) after a radical polymerization initiator is added thereto. It is noted herein that two or more types of initiators may be combined to arrive at a more controlled and completed polymerization reaction.

According to some embodiments of the present invention, the initiation step is effected at relatively low temperatures as a function of the choice of initiator. According to some embodiments of the present invention, the co-polymeric composition includes a low temperature dissociation initiator which keeps the formed multifocal ophthalmic device fixed in position in the mold by avoiding significant expansion or contraction. Using such initiators makes the use of fused silica molds redundant, which in turn reduces the need for complex UV curing of the composition. Hence, according to some embodiments of the present invention, the initiator is a low temperature dissociation initiator.

According to some embodiments of the present invention, non-limiting examples of the initiator include bedicetyl peroxydicarbonate, tert-butyl peroxypivalate, diisobutyryl peroxide, dimyristyl peroxydicarbonate, 1,1,3,3-tetramethylbutyl peroxypivalate, tert-butyl peroxyneoheptanoate, di(2-neodecanoylperoxy-isopropyl)benzene, cumylperoxy-neodecanoate, 1,1,3,3-tetramethylbutylperoxy-neodecanoate, t-butylperoxy-neodecanoate, t-butylperoxy-neoheptanoate and any combinations thereof.

According to other embodiments of the present invention, the catalyst is selected from the group consisting of dicetyl peroxydicarbonate (such as Perkadox® 24L by Akzo Nobel Polymer Chemicals, India) and tert-butyl peroxypivalate (such as LUPEROX® 554M75 by Arkema Inc. Philadelphia, PA, USA).

Non-limiting examples of a radical and/or thermal polymerization initiator include, for instance, azobisisobutyronitorile, azobisdimethylvaleronitrile, benzoyl peroxide, tert-butyl hydroperoxide, qumene hydroperoxide and the like, which can be used solely or in a combination use of two or more thereof.

Light-sensitive initiators (photopolymerization initiator) include, for a non-limiting example, photopolymerization initiators of benzoin compounds such as methyl orthobenzoyl benzoate, methyl benzoyl formate, benzoin methyl ether, benzoin ethyl ether, benzoin isopropyl ether, benzoin isobutyl ether and benzoin n-butyl ether, photopolymerization initiators of phenone compounds such as 2-hydroxy-2-methyl-1-phenylpropane-1-one, p-isopropyl-$\alpha$-hydroxyisobutylphenone, p-tert-butyltrichloroacetophenone, 2,2-dimethoxy-2-phenylacetophenone, $\alpha,\alpha$-dichloro-4-phenoxyacetophenone, and N,N-tetraethyl-4,4-diaminobenzophenone, 1-hydroxycyclohexylphenylketone, 1 -phenyl-1,2-propanedione-2-(o-ethoxycarbonyl) oxime, photopolymerization initiators of thioxanthone such as 2-chlorothioxanthone and 2-methylthioxanthone, dibenzosuberone, 2-ethylanthraquinone, benzophenone acrylate, benzophenone, benzil and the like.

The amount of the above-mentioned polymerization initiator is typically not less than 0.002 percent by weight, more preferably 0.01 % by weight based on 100 % of the total weight of the composition. Alternatively, the concentration of the initiator is not more than 10 % by weight, or not more than 2 % of the total weight of the composition.

The optional addition of initiator (also referred to herein interchangeably as a catalyst) prior to the casting stage, is meant to boost the completion of the polymerization reaction. As discussed hereinabove, additional initiator is added when the viscosity of the reaction mixture reaches about 120 cps.

According to some embodiments of the present invention, the process includes first adding an amount equivalent to about 20-40 % of the total amount of the initiator in the first step of the process, and then adding about 60-80 % of the total weight of the initiator.

After mixing and adding the initiator, the mixture is transferred through a filter into the mold. The viscosity at which the mixture is filtered is about 120-130 cps. The ophthalmic device is formed in the mold from the mixture directly into its complete form, avoiding further machining which in turn avoids local burning and machining related stresses. After casting or pouring the mixture in between two halves of the mold, thermal or photo curing stimulus is applied. Thermal curing is performed at room temperature to 80 °C with several ramping steps.

According to some embodiments of the present invention, filtering the reaction mixture may also precede the casting stage.

Once the reaction mixture is cast in a mold, the curing step can start. Exposure to curing conditions typically includes elevating the temperature and/or exposing the reaction mixture to high energy radiation. High temperatures are typically about 80 °C, and the irradiation energy may range from 10 KJ/Kg to 50 KJ/Kg in order to further allow the co-polymeric composition to cure.

Curing agents and accelerators may also be employed in the formation of the co-polymeric composition according to some embodiments of the present invention. Various curing agents and accelerators are known and can be used in prescribed amounts or amounts experimentally found to be suitable. Typically, amounts of the curing agent, the curing agent accelerator or a combination thereof are between about 0.1 % and about 8 % by weight of the total weight of the composition. Curing agents and accelerators can be used in various amounts, which will typically depend upon the monomers and polymers being employed, any ambient conditions (e.g., heat, light or otherwise) being used for curing and/or other factors.

Examples of suitable curing agents include UV photoinitatiators, peroxy catalysts (i.e., any catalyst including a peroxy group), oxide catalysts (i.e., any catalyst include an oxide group (e.g., a dioxide) or others known by the skilled artisan. One example of a peroxy catalyst is a tert-butyl peroxy-2-ethylhexanoate organic peroxide initiator, which is particularly suitable for thermal cure. One example of an oxide catalyst is 2,4,6-trimethylbenzoyldiphenylphosphine oxide, which is particularly suitable for blue light cure.

According to some embodiments of the present invention, the monomers, the initiator, the curing agent and optionally curing agent accelerator, a UV-blocker (radiation resistant compound), if present, and any other desired ingredients are combined together to form a master batch. The master batch is then exposed to an ambient stimulus, such as heat or light (e.g., blue light) which initiates polymerization and cross-linking reactions between the various monomers. The initiated master batch can be cast into molds (such as cast wafers) of desired geometry and can be secured in cure fixtures for forming the ophthalmic devices. It is advisable to add a crosslinking monomer in initial stage especially in the case where the polymerization reaction is performed at relatively low temperature.

The wafer molds are then typically cured through extended exposure to an ambient condition such as heat, light or both. For example, in one embodiment, the cast wafers are exposed to an elevated temperature (e.g., about 70 °C) for a first period of time (e.g., about 2 hours) and then ramped up to a second temperature (e.g., about 110°C) for a second period of time (e.g., at least 10 minutes). In a second exemplary embodiment, the wafers are cured using blue light at a wavelength of about 405 nm to about 415 nm for a first period of time (e.g., about 3 hours) and then exposed to an elevated temperature (e.g., about 110 °C) for a second period of time (e.g., about one hour). Typically, the initiation, the curing or both are carried out in a low moisture (e.g., less than 1 ppm water) and low oxygen (less than 100 ppm) environment.

Alternatively, working at relatively low temperatures, from room temperature and up to 80 °C is advantageous, since it allows the polymerization and curing steps to complete post the casting stage (namely in the mold), without causing thermal distortion of the mold. This way a relatively low cost polypropylene mold can be used, instead of a costlier fused silica molds.

During the initial polymerization step it is possible to monitor the viscosity of the master batch mixture, for example by following the force applied to the mixing shaft.

Once the polymeric or co-polymeric composition is cured it can be cleansed from unreacted components and other leachables. The extraction of leachables may commence once the molded and cured composition is released from the mold. According to some embodiments of the present invention, the extraction is effected by sequential immersion of the polymeric or co-polymeric composition in a series of baths, each containing a different solvent or solution, going from hydrophobic to hydrophilic in the order of sequence, thereby extracting unreacted contaminants from the composition.

Following the description of the embodiments of the present invention, provides co-polymeric compositions and processes for preparing the same, which are highly suitable for manufacturing implantable and non-implantable ophthalmic and ocular devices including multifocal ophthalmic devices. Following the above-described description of the embodiments affords vacuole free, glistening free, internal reflection free and tack free ophthalmic and ocular devices, which meet the requirement of tensile strength, deformation recovery ability and mechanical, optical, biological and toxicological requirements set by widely accepted standards of the art.

It is noted that even after the post-curing step, which further pushes the polymerization reaction to completion, some impurities from unreacted monomers and other contaminants remains in the composition. These impurities are commonly referred to herein as unreacted components, diluents and other leachable impurities. Under the term leachable impurities are also included filter membrane residues, boiling impurities, solvent remnants and other process contaminants.

To remove unreacted components and diluents (UCDs) and other leachable impurities from the cured composition formed as a multifocal ophthalmic device, and affect clinical viability of the lens body, the process of preparing such devices typically includes an extraction step. If the leachable substances are not extracted from the device, they may make the device uncomfortable to wear or even present a medical hazard. As used herein, "leachable substance"

includes UCDs and other substances which are not bound to or embedded in the polymer and may be extracted from the composition (the matrix), for example, by leaching with water or an organic solvent. As used herein, the term "treat" means to expose a cured object or device, made from the composition presented herein, to an aqueous and/or organic solution which may also include at least one leaching aid. Treating a cured polymeric composition to remove UCDs and monitoring traces thereof is demonstrated in the Examples section which follows below using a curcuminoid compound as a light-stabilizer, which is not part of the polymeric backbone and therefore required not to leach out of the cured composition.

As used herein, a "leaching aid" is any compound that if used in an effective amount in an aqueous or organic solution to treat an ophthalmic device, and can assist in obtaining a device with an adequate amount of removal of leachable substances.

According to embodiments of the present invention, the process of preparing the lens body of a multifocal ophthalmic device from the co-polymeric composition presented herein may include a treatment of the cured co-polymeric composition. The treatment step can include exposing the cured composition to an aqueous and/or organic solution which constitutes or includes at least one leaching aid. In various embodiments, treatment can be accomplished, for example, via immersion of the device in a solution or exposing the device to a flow of solution or exposing the device to Soxhlet extraction. In various embodiments, treatment can also include, for example, one or more of heating the solution; stirring the solution; mechanical agitation or sonication of the solution; and increasing the level of leach aid in the solution to a level sufficient to facilitate adequate removal of leachable substances from the device.

According to some embodiments of the present invention, an organic or aqueous solution may constitute a leaching aid. According to other embodiments of the present invention, leaching aids can also be combined with organic solvents to improve the rate of release. For example, in some embodiments, ophthalmic devices such as lenses can be subjected to a treatment exposing the lens devices to a leaching aid and a GC Mass Spectrometer can be used to measure the level of one or more leachable substances in the lens devices. The GC Mass Spectrometer can determine whether treatment with a particular leaching aid is effective to reduce an amount of particular leachable substances present in the lenses to a maximum threshold amount. Accordingly, in some embodiments, a GC Mass Spectrometer can be used to check for a maximum threshold of leachable substances of approximately 300 ppm. A minimum hydration treatment time period necessary to reduce the presence of such leachable substances to 300 ppm or less in specific lenses can be determined by the periodic measurements. In additional embodiments, other leachable substances, such as, for example, D3O or other diluents, can be measured to detect the presence of a maximum amount of approximately 60 ppm. Embodiments can also include setting a threshold amount of a particular leachable substance at the minimum detection level ascertainable by the testing equipment.

Examples of leaching aids, according to the present invention include, without limitations, alkanes, ketones (e.g. 2-butanone), amides, ethers (e.g. THF), alcohols (e.g. methanol), esters (e.g. ethyl acetate), aldehydes, nitrogen-containing cyclic compounds, toluene, water, ethoxylated alcohols or ethoxylated carboxylic acids, ethoxylated glucosides or sugars, optionally with attached C6-Cl4 carbon chains, polyalkylene oxides, sulfates, carboxylates or amine oxides of C6-Cl4 compounds. Examples include cocamidopropylamine oxide, C6-Cl4 fatty alcohol ethoxylated with ethylene oxides, sodium dodecyl sulfate, polyoxyethylene-2-ethyl hexyl ether, polypropylene glycol, polyethylene glycol monomethyl ether, ethoxylated methyl glucoside dioleate, and the sodium salt of n-octylsulfate, sodium salt of ethylhexyl sulfate.

By way of non-limiting examples, various implementations can include release and removal of leachable impurities that is accomplished by way of a batch process wherein devices are submerged in a solution contained in a fixed tank for a specified period of time or in a vertical process where devices are exposed to a continuous flow of a solution that includes at least one of a leach aid. In some embodiments, the solution can be heated with a heat exchanger or other heating apparatus to further facilitate leaching of the device. For example, heating can include raising the temperature of an aqueous or organic solution to the boiling point while a device is submerged in the heated solution. Other embodiments can include controlled cycling of the temperature of the solution. Some embodiments can also include the application of physical agitation to facilitate leach. For example, a strainer container holding the device can be vibrated or caused to move back and forth within a leaching solution. Other embodiments may include ultrasonic waves through the solution.

The choice of a leaching aid or solvent depends on the impurities present in the composition. Each solvent has a capability of extracting certain impurities with an overlapping range of chemical efficiency. For example, 2-butanone, THF, methanol and ethyl acetate are used sequentially on a same set of fully cured devices; which helps in removing all identified impurities step by step. Water miscible solvents are kept for last. A continuous soxhlet extraction may be employed which utilizes fresh solvent each time. Each soxhlet extracting phase may be sized to hold 500 lenses of 20 diopter. Maximum quantity varies with the power for given sized extractor. However extractors can be easily scaled up to meet the quantity requirement. Flow rate for the solvent varies according to extractor and according to cycle time. A holding time of 3 hours is kept for each solvent. For example, a drop rate of 100 ml/hr is kept for 300 ml extractor.

For example, freshly cured multifocal ophthalmic devices, prepared from the co-polymeric composition presented herein are kept in glass thimble, which is soaked in a bath of one leaching aid solution, and then exchanges its position to a bath holding the next solvent and so on; wherein each soak is maintained for 15-30 minutes. A vacuum tempering is performed thereafter in order to dry the devices at 110 °C and 0.1 mbar.

According to some embodiments of the present invention, post operation of reducing tack may be performed on a anterior surface or on both surface by processing lens by methods such as plasma treatment, surface fluorination, bulk fluorination, hydrophilic coating, irradiating with EB rays, high energy UV rays or by other energy intensive rays, use of internal wetting agents for selective migration and allied.

It should be noted herein that according to some embodiments of the present invention, the composition presented herein can be used to manufacture ophthalmic devices as described herein which are used also for drug delivery. In these embodiments, the non-leachability of the main additives and components (such as, for example, the curcuminoid compound discussed hereinbelow) is in effect, however, the drug which is delivered from the device to the surrounding tissue is in fact leachable, and can diffuse from the matrix (typically a hydrogel) to the physiological medium in which the device is situated and from there to the tissue to be treated.

An ophthalmic or ocular device, typically a lens, and particularly the lens body of a multifocal ophthalmic device as presented hereinabove, formed from the composition presented herein, can be manufactured by using one or two basic methods: molding into a final form without further machining, and shaping and molding followed by machining for reshaping and polishing. Hence, a device as described herein, made from a polymeric or co-polymeric composition as presented herein, can be made while preparing the polymeric or co-polymeric composition, or prepared from a pre-formed polymeric or co-polymeric composition.

For example, multi-part molds can be used to fashion the composition presented hereinbelow into a useful article of a complex shape, such as the lens body of the multifocal ophthalmic device presented hereinabove or any ophthalmic lens. In the case of the lens body of a multifocal ophthalmic device as presented herein, the multi-part molds can include for example, a first mold part with a convex or concave surface that corresponds to a back curve of an ophthalmic lens, and a second mold portion with a generally convex surface that corresponds to the complex structure of the front curve of the multifocal lens, which includes an inverse form of the plurality of concentric annular zones separated by slanted steps, as described herein. To prepare a lens using such mold portions, the uncured lens' composition is placed between the mold portions and subsequently cured. The lens' composition may then be cured, for example by exposure to either or both heat and light. The cured composition forms a lens body according to the dimensions and features of the mold portions. Following curing, traditional practice dictates that the mold portions are separated and the lens remains adhered to one of the mold portions, calling for a release process to detach the lens from the remaining mold part. In some embodiments, the process of manufacturing the lens body, the cured lens body having the plurality of concentric annular zones separated by slanted steps may be subjected to further polishing and/or shaping to achieve final desired form.

The formation of the ophthalmic device from a polymeric or co-polymeric composition as presented herein can also be effected by, for example, a computer-controllable manufacturing device. For example, the lens body of the multifocal ophthalmic device presented herein may be shaped into final form in two basic steps, wherein in the first step the crude lens is forged from the co-polymeric composition presented herein, and in the second step the plurality of concentric annular zones separated by slanted steps, as described herein, are formed in the cured crude lens body using a computer controllable manufacturing device.

It is expected that during the life of a patent maturing from this application many relevant intra-occular lens methods, materials and constructions will be developed and the scope of the term 'intra-ocular lens' is intended to include all such new technologies a priori.

The terms "comprises", "comprising", "includes", "including", "having" and their conjugates mean "including but not limited to".

The term "consisting of" means "including and limited to".

As used herein, the singular form "a", "an" and "the" include plural references unless the context clearly dictates otherwise.

It is appreciated that certain features of the invention, which are, for clarity, described in the context of separate embodiments, may also be provided in combination in a single embodiment, and the above description is to be construed as if this combination were explicitly written. Conversely, various features of the invention, which are, for brevity, described in the context of a single embodiment, may also be provided separately or in any suitable subcombination or as suitable in any other described embodiment of the invention, and the above description is to be construed as if these separate embodiments were explicitly written. Certain features described in the context of various embodiments are not to be considered essential features of those embodiments, unless the embodiment is inoperative without those elements.

## Claims

1. An intraocular lens comprising:

    a lens body (100) customized for astigmatism in a particular eye, the customization requiring an alignment following insertion within the eye;
    a plurality of protrusions (114, 116, 118, 120) arranged outwardly around the lens body (100) to form a substantially rectangular profile, thereby to lock said intraocular lens into a position of rotational stability when inserted into a capsular bag, thus to prevent rotation of said intraocular lens after insertion into said capsular bag, wherein the intraocular lens comprises a cylindrical value for treatment of astigmatism, wherein said protrusions (114, 116, 118, 120) comprise protrusions of haptic pads (106, 108);
    **characterized in that:**

        said haptic pads (106, 108) are hinged to said lens body (100); and
        a width (110) of said haptic pads (106, 108) is substantially the same as an optical diameter (112) of the lens (100).

2. The intraocular lens of claim 1, wherein said protrusions (114, 116, 118, 120) form rounded corners of a rectangle around said lens body (100).

3. The intraocular lens of claim 1, wherein said haptic pads comprise resilient material, or wherein said haptic pads (106, 108) comprise a cross-sectional profile which is thinner than that of said lens body (100), or wherein said haptic pads (106, 108) are foldable.

4. The intraocular lens of any one of the preceding claims, further comprising orientation markers (126, 128) to distinguish a top of said lens body (100) from a bottom of said lens body (100).

5. The intraocular lens according to claim 1, comprising: axis markers to distinguish an optical axis of said intraocular lens, wherein said axis markers comprise two groups of three holes respectively at different ends of said axis, said groups distanced from an edge of said lens body (100) by a predetermined margin.

6. The intraocular lens of any one of the preceding claims, comprising an overall length along a diagonal of substantially rectangular shape, said overall length being customized according to an estimated size of a respective capsular bag.

7. The intraocular lens of any one of the preceding claims, having a profile to treat astigmatism, the profile being one member of the group consisting of:

    a cylindrical profile;
    a toric profile (134); and
    an aspherical profile (138).

8. The intraocular lens of claim 7, wherein astigmatism of said lens is aligned with an optical axis of said lens and said optical axis is marked with axis markers (130, 132).

9. The intraocular lens of any one of the preceding claims, further comprising a diffractive lens (144).

10. The intraocular lens of claim 9, wherein said diffractive lens (144) provides a near focus and a far focus, thereby to provide a bifocal intraocular lens.

11. The intraocular lens of any one of the preceding claims, said lens body (100) having two opposite sides, wherein a first side has an aspheric profile and a diffractive pattern formed thereon, and said second side has a toric profile.

12. The intraocular lens of claim 11, wherein said toric profile is devoid of spherical aberration and wherein a cylinder value characterizing said lens body (100) is at least 1 Diopter, or wherein said diffractive pattern comprises a plurality of concentric annular zones having surfaces separated by slanted steps having surfaces, wherein said surfaces of said concentric zones effect both diffraction and refraction of incident light, while said surfaces of said steps are substantially devoid of any diffractive or refractive power.

**13.** The intraocular lens of claim 11, wherein said diffractive pattern comprises a plurality of concentric annular zones having surfaces separated by slanted steps having surfaces, wherein said surfaces of said concentric zones effect both diffraction and refraction of incident light, while said surfaces of said steps are substantially devoid of any diffractive or refractive power and each step of said steps is **characterized by** a radius, a slope and a height, and wherein said slope and said height are at least non-increasing functions of said radius.

**14.** The intraocular lens of any one of the preceding claims, wherein said lens body (100) is one member of the group consisting of:

a lens body made of biocompatible material;
a lens body comprising a hydrophilic acrylic material;
a lens body comprising a polymeric or co-polymeric composition, and at least one curcuminoid compound incorporated in or on said polymeric or co-polymeric composition and/or said lens body;
a lens body comprising a co-polymeric composition having a polymeric backbone composed of a plurality of backbone units covalently linked to one another, said backbone units being derived from a pre-polymerization mixture of monomers; and
a lens body comprising a polymeric or co-polymeric composition derived from a pre-polymerization mixture of monomers which comprises at least 50 weight percents acrylate monomers.

**15.** A method of forming an intraocular lens, comprising:

obtaining an estimate of a length of a capsular bag of an eye requiring said intraocular lens;
forming a lens body (100) having a first side and a second side, customizing said lens with astigmatism aligned along an optical axis to match an astigmatism of said eye, said customizing comprising providing a toric profile being devoid of spherical aberration wherein a cylinder value characterizing said lens body is at least 1 Diopter;
providing haptic pads (106, 108) around said lens body (100), said haptic pads being shaped to provide a substantially rectangular profile to surround said optical axis, said optical axis being aligned along said rectangular profile,
**characterized in that:**

said estimate of a length being used to determine a size of said intraocular lens by defining a diagonal of said rectangular profile,
said haptic pads (106, 108) are hinged to said lens body (100), and
a width (110) of said haptic pads (106, 108) is substantially the same as an optical diameter (112) of the lens (100).

## Patentansprüche

**1.** Intraokularlinse, umfassend:

einen Linsenkörper (100), der für Astigmatismus in einem bestimmten Auge angepasst ist, wobei die Anpassung eine Ausrichtung nach dem Einsetzen in das Auge erfordert;
eine Vielzahl von Vorsprüngen (114, 116, 118, 120), die nach außen um den Linsenkörper (100) herum angeordnet sind, um ein im Wesentlichen rechteckiges Profil zu bilden, um dadurch die Intraokularlinse in einer drehstabilen Position zu blockieren, wenn sie in einen Kapselsack eingesetzt wird, um somit eine Drehung der Intraokularlinse nach dem Einsetzen in den Kapselsack zu verhindern, wobei die Intraokularlinse einen Wert des Zylinders zur Behandlung von Astigmatismus umfasst, wobei die Vorsprünge (114, 116, 118, 120) Vorsprünge von haptischen Blöcken (106, 108) umfassen;
**dadurch gekennzeichnet, dass**:

die haptischen Blöcke (106, 108) an dem Linsenkörper (100) angelenkt sind; und
eine Breite (110) der haptischen Blöcke (106, 108) im Wesentlichen gleich einem optischen Durchmesser (112) der Linse (100) ist.

**2.** Intraokularlinse nach Anspruch 1, wobei die Vorsprünge (114, 116, 118, 120) die abgerundeten Ecken eines Rechtecks um den Linsenkörper (100) herum bilden.

**3.** Intraokularlinse nach Anspruch 1, wobei die haptischen Blöcke ein federelastisches Material umfassen, oder wobei die haptischen Blöcke (106, 108) ein Querschnittsprofil umfassen, das dünner als das des Linsenkörpers (100) ist, oder wobei die haptischen Blöcke (106, 108) faltbar sind.

**4.** Intraokularlinse nach einem der vorhergehenden Ansprüche, ferner umfassend Orientierungsmarkierungen (126, 128), um einen oberen Teil des Linsenkörpers (100) von einem unteren Teil des Linsenkörpers (100) auseinanderzuhalten.

**5.** Intraokularlinse nach Anspruch 1, umfassend: Achsenmarkierungen, um eine optische Achse der Intraokularlinse auseinanderzuhalten, wobei die Achsenmarkierungen zwei Gruppen von drei Löchern jeweils an verschiedenen Enden der Achse umfassen, wobei die Gruppen von einem Rand des Linsenkörpers (100) um einen vorbestimmten Randabstand beabstandet sind.

**6.** Intraokularlinse nach einem der vorhergehenden Ansprüche, umfassend eine Gesamtlänge entlang einer Diagonalen von im Wesentlichen rechteckiger Form, wobei die Gesamtlänge gemäß einer geschätzten Größe eines jeweiligen Kapselsacks angepasst ist.

**7.** Intraokularlinse nach einem der vorhergehenden Ansprüche, die ein Profil aufweist, um Astigmatismus zu behandeln, wobei das Profil zu der Gruppe gehört, die besteht aus:

einem zylindrischen Profil;
einem torischen Profil (134); und
einem asphärischen Profil (138).

**8.** Intraokularlinse nach Anspruch 7, wobei der Astigmatismus der Linse auf eine optische Achse der Linse ausgerichtet ist und die optische Achse mit Achsenmarkierungen (130, 132) markiert ist.

**9.** Intraokularlinse nach einem der vorhergehenden Ansprüche, ferner umfassend eine diffraktive Linse (144).

**10.** Intraokularlinse nach Anspruch 9, wobei die diffraktive Linse (144) einen Nahfokus und einen Fernfokus bereitstellt, um dadurch eine bifokale Intraokularlinse bereitzustellen.

**11.** Intraokularlinse nach einem der vorhergehenden Ansprüche, wobei der Linsenkörper (100) zwei gegenüberliegende Seiten aufweist, wobei auf einer ersten Seite ein asphärisches Profil und ein diffraktives Muster gebildet sind und die zweite Seite ein torisches Profil aufweist.

**12.** Intraokularlinse nach Anspruch 11, wobei das torische Profil von sphärischen Aberrationen frei ist, und wobei ein Wert des Zylinders, der den Linsenkörper (100) charakterisiert, mindestens 1 Diopter beträgt, oder wobei das diffraktive Muster eine Vielzahl von konzentrischen ringförmigen Zonen umfasst, die Oberflächen aufweisen, die durch schräge Stufen, die Oberflächen aufweisen, getrennt sind, wobei sich die Oberflächen der konzentrischen Zonen sowohl auf die Diffraktion als auch auf die Refraktion von einfallendem Licht auswirken, während die Oberflächen der Stufen im Wesentlichen frei von diffraktiver oder refraktiver Kraft sind.

**13.** Intraokularlinse nach Anspruch 11, wobei das diffraktive Muster eine Vielzahl von konzentrischen ringförmigen Zonen umfasst, die Oberflächen aufweisen, die durch schräge Stufen, die Oberflächen aufweisen, getrennt sind, wobei sich die Oberflächen der konzentrischen Zonen sowohl auf die Diffraktion als auch auf die Refraktion von einfallendem Licht auswirken, während die Oberflächen der Stufen im Wesentlichen frei von diffraktiver oder refraktiver Kraft sind, und jede Stufe der Stufen durch einen Radius, eine Neigung und eine Höhe charakterisiert ist, und wobei die Neigung und die Höhe mindestens nicht zunehmende Funktionen des Radius sind.

**14.** Intraokularlinse nach einem der vorhergehenden Ansprüche, wobei der Linsenkörper (100) zu der Gruppe gehört, die besteht aus:

einem Linsenkörper aus biokompatiblem Material;
einem Linsenkörper, der ein hydrophiles Acrylmaterial umfasst;
einen Linsenkörper, der eine Polymer- oder Copolymer-Zusammensetzung und mindestens eine Curcuminoid-Zusammensetzung, die in oder auf der Polymer- oder Copolymer-Zusammensetzung und/oder den Linsenkörper eingearbeitet ist, umfasst;

einen Linsenkörper, der eine Copolymer-Zusammensetzung umfasst, die ein Polymer-Rückgrat aufweist, das aus einer Vielzahl von Rückgrateinheiten besteht, die kovalent miteinander verbunden sind, wobei die Rückgrateinheiten von einer Vorpolymerisationsmischung von Monomeren abgeleitet werden; und
einen Linsenkörper, der eine Polymer- oder Copolymer-Zusammensetzung umfasst, die aus einer Vorpolymerisationsmischung von Monomeren abgeleitet wird, die mindestens 50 Gewichtsprozent Acrylat-Monomere umfasst.

**15.** Verfahren zum Bilden einer Intraokularlinse, umfassend:

Erzielen einer Schätzung einer Länge eines Kapselsacks eines Auges, das die Intraokularlinse benötigt;
Bilden eines Linsenkörpers (100), der eine erste Seite und eine zweite Seite aufweist, Anpassen der Linse mit Astigmatismus, die entlang einer optischen Achse ausgerichtet ist, um mit einem Astigmatismus des Auges übereinzustimmen, wobei das Anpassen das Bereitstellen eines torischen Profils, das frei von sphärischen Aberrationen ist, umfasst, wobei ein Wert des Zylinders, der den Linsenkörper charakterisiert, mindestens 1 Diopter beträgt;
Bereitstellen von haptischen Blöcken (106, 108) um den Linsenkörper (100) herum, wobei die haptischen Blöcke gestaltet sind, um ein im Wesentlichen rechteckiges Profil bereitzustellen, um die optische Achse zu umgeben, wobei die optische Achse entlang des rechteckigen Profils ausgerichtet ist,
**dadurch gekennzeichnet, dass**:

die Schätzung einer Länge verwendet wird, um eine Größe der Intraokularlinse durch Definieren einer Diagonale des rechteckigen Profils zu bestimmen,
die haptischen Blöcke (106, 108) an dem Linsenkörper (100) angelenkt sind, und
eine Breite (110) der haptischen Blöcke (106, 108) im Wesentlichen gleich einem optischen Durchmesser (112) der Linse (100) ist.

## Revendications

**1.** Lentille intraoculaire comprenant :

un corps de lentille (100) adapté à l’astigmatisme dans un oeil particulier, la personnalisation nécessitant un alignement après insertion dans l’oeil ;
une pluralité de saillies (114, 116, 118, 120) disposées vers l’extérieur autour du corps de lentille (100) pour former un profil sensiblement rectangulaire, afin de verrouiller ladite lentille intraoculaire dans une position de stabilité en rotation lorsqu’elle est insérée dans un sac capsulaire, afin d’empêcher ainsi la rotation de ladite lentille intraoculaire après insertion dans ledit sac capsulaire, dans laquelle la lentille intraoculaire comprenant une valeur cylindrique pour le traitement de l’astigmatisme, lesdites saillies (114, 116, 118, 120) comprenant des saillies de coussinets haptiques (106, 108) ;
**caractérisée en ce que** :

lesdits coussinets haptiques (106, 108) sont articulés sur ledit corps de lentille (100) ; et
qu’une largeur (110) desdits coussinets haptiques (106, 108) est sensiblement la même qu’un diamètre optique (112) de la lentille (100).

**2.** Lentille intraoculaire selon la revendication 1, dans laquelle lesdites saillies (114, 116, 118, 120) forment les coins arrondis d’un rectangle autour dudit corps de lentille (100).

**3.** Lentille intraoculaire selon la revendication 1, dans laquelle lesdits coussinets haptiques comprennent un matériau élastique, ou dans laquelle lesdits coussinets haptiques (106, 108) comprennent un profil en coupe transversale qui est plus mince que celui dudit corps de lentille (100), ou dans laquelle lesdits coussinets haptiques (106, 108) sont pliables.

**4.** Lentille intraoculaire selon l’une quelconque des revendications précédentes, comprenant en outre des marqueurs d’orientation (126, 128) pour distinguer un sommet dudit corps de lentille (100) d’un fond dudit corps de lentille (100).

**5.** Lentille intraoculaire selon la revendication 1, comprenant : des marqueurs d’axe pour distinguer un axe optique de ladite lentille intraoculaire, lesdits marqueurs d’axe comprenant deux groupes de trois trous respectivement à des

extrémités différentes dudit axe, lesdits groupes étant éloignés d'un bord dudit corps de lentille (100) à raison d'une marge prédéterminée.

6. Lentille intraoculaire selon l'une quelconque des revendications précédentes, comprenant une longueur totale le long d'une diagonale de forme sensiblement rectangulaire, ladite longueur totale étant personnalisée selon une taille estimée d'un sac capsulaire correspondant.

7. Lentille intraoculaire selon l'une quelconque des revendications précédentes, ayant un profil pour traiter l'astigmatisme, le profil étant un élément du groupe constitué par :

   un profil cylindrique ;
   un profil torique (134) ; et
   un profil asphérique (138).

8. Lentille intraoculaire selon la revendication 7, dans laquelle l'astigmatisme de ladite lentille est aligné avec un axe optique de ladite lentille et ledit axe optique est marqué par des marqueurs d'axe (130, 132).

9. Lentille intraoculaire selon l'une quelconque des revendications précédentes, comprenant en outre une lentille diffractive (144).

10. Lentille intraoculaire selon la revendication 9, dans laquelle ladite lentille diffractive (144) crée un foyer proche et un foyer lointain, afin de créer ainsi une lentille intraoculaire bifocale.

11. Lentille intraoculaire selon l'une quelconque des revendications précédentes, ledit corps de lentille (100) ayant deux côtés opposés, un premier côté ayant un profil asphérique et un motif diffractif formé sur celui-ci, et ledit second côté ayant un profil torique.

12. Lentille intraoculaire selon la revendication 11, dans laquelle ledit profil torique est dépourvu d'aberration sphérique et dans laquelle une valeur de cylindre caractérisant ledit corps de lentille (100) est d'au moins 1 dioptrie, ou dans laquelle ledit motif diffractif comprend une pluralité de zones annulaires concentriques ayant des surfaces séparées par des échelons inclinés présentant des surfaces, lesdites surfaces desdites zones concentriques effectuant à la fois la diffraction et la réfraction de la lumière incidente, tandis que lesdites surfaces desdits échelons sont sensiblement dépourvues de tout pouvoir diffractif ou réfractif.

13. Lentille intraoculaire selon la revendication 11, dans laquelle ledit motif diffractif comprend une pluralité de zones annulaires concentriques ayant des surfaces séparées par des échelons inclinés présentant des surfaces, lesdites surfaces desdites zones concentriques effectuant à la fois la diffraction et la réfraction de la lumière incidente, tandis que lesdites surfaces desdits échelons sont sensiblement dépourvues de tout pouvoir diffractif ou réfractif et que chaque échelon parmi lesdits échelons est **caractérisé par** un rayon, une pente et une hauteur, ladite pente, et ladite pente et ladite hauteur étant au moins des fonctions non croissantes dudit rayon.

14. Lentille intraoculaire selon l'une quelconque des revendications précédentes, dans laquelle ledit corps de lentille (100) est un élément du groupe constitué par :

   un corps de lentille en matériau biocompatible ;
   un corps de lentille comprenant un matériau acrylique hydrophile ;
   un corps de lentille comprenant une composition polymérique ou copolymérique, et au moins un composé curcuminoïde incorporé dans ou sur ladite composition polymérique ou copolymérique et/ou ledit corps de lentille ;
   un corps de lentille comprenant une composition copolymérique ayant une ossature polymérique composée d'une pluralité d'unités d'ossature liées de manière covalente les unes aux autres, lesdites unités d'ossature étant dérivées d'un mélange de monomères de prépolymérisation ; et
   un corps de lentille comprenant une composition polymérique ou copolymérique dérivée d'un mélange de prépolymérisation de monomères qui comprend au moins 50 % en poids de monomères acrylates.

15. Procédé de formation d'une lentille intraoculaire, comprenant :

   l'obtention d'une estimation de la longueur d'un sac de capsule d'un oeil nécessitant ladite lentille intraoculaire ;

la formation d'un corps de lentille (100) ayant un premier côté et un second côté, la personnalisation de ladite lentille avec un astigmatisme aligné le long d'un axe optique pour correspondre à un astigmatisme dudit oeil, ladite personnalisation comprenant la création d'un profil torique dépourvu d'aberration sphérique, une valeur de cylindre caractérisant ledit corps de lentille étant d'au moins 1 dioptrie ;
la prévision de coussinets haptiques (106, 108) autour dudit corps de lentille (100), lesdits coussinets haptiques étant conformés pour créer un profil sensiblement rectangulaire pour entourer ledit axe optique,
ledit axe optique étant aligné le long dudit profil rectangulaire,
**caractérisé en ce que** :

ladite estimation d'une longueur est utilisée pour déterminer une taille de ladite lentille intraoculaire en définissant une diagonale dudit profil rectangulaire,
lesdits coussinets haptiques (106, 108) sont articulés sur ledit corps de lentille (100), et
une largeur (110) desdits coussinets haptiques (106, 108) est sensiblement la même qu'un diamètre optique (112) de la lentille (100).

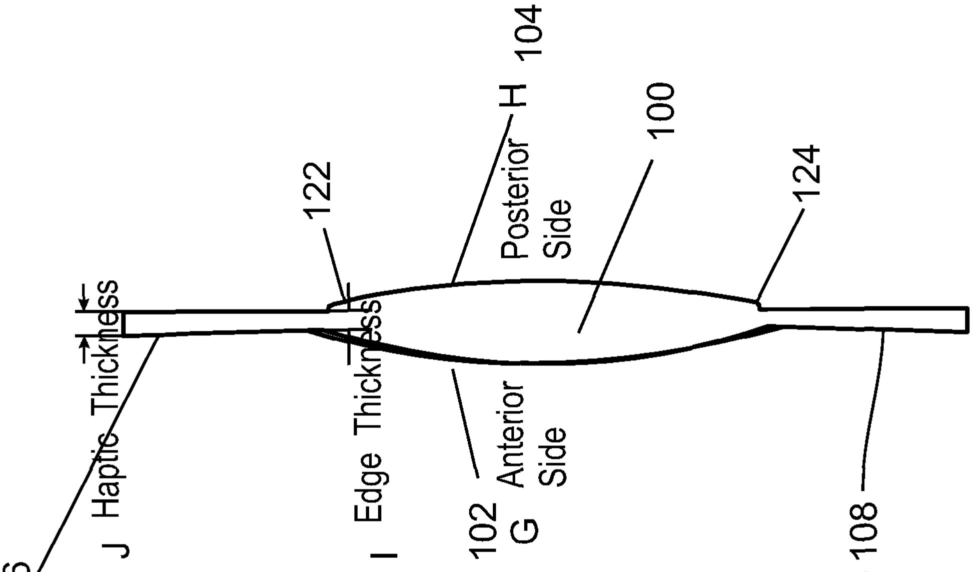
106
J Haptic Thickness
I Edge Thickness
102 G Anterior Side
122
Posterior Side
H 104
100
124
108

FIG. 1

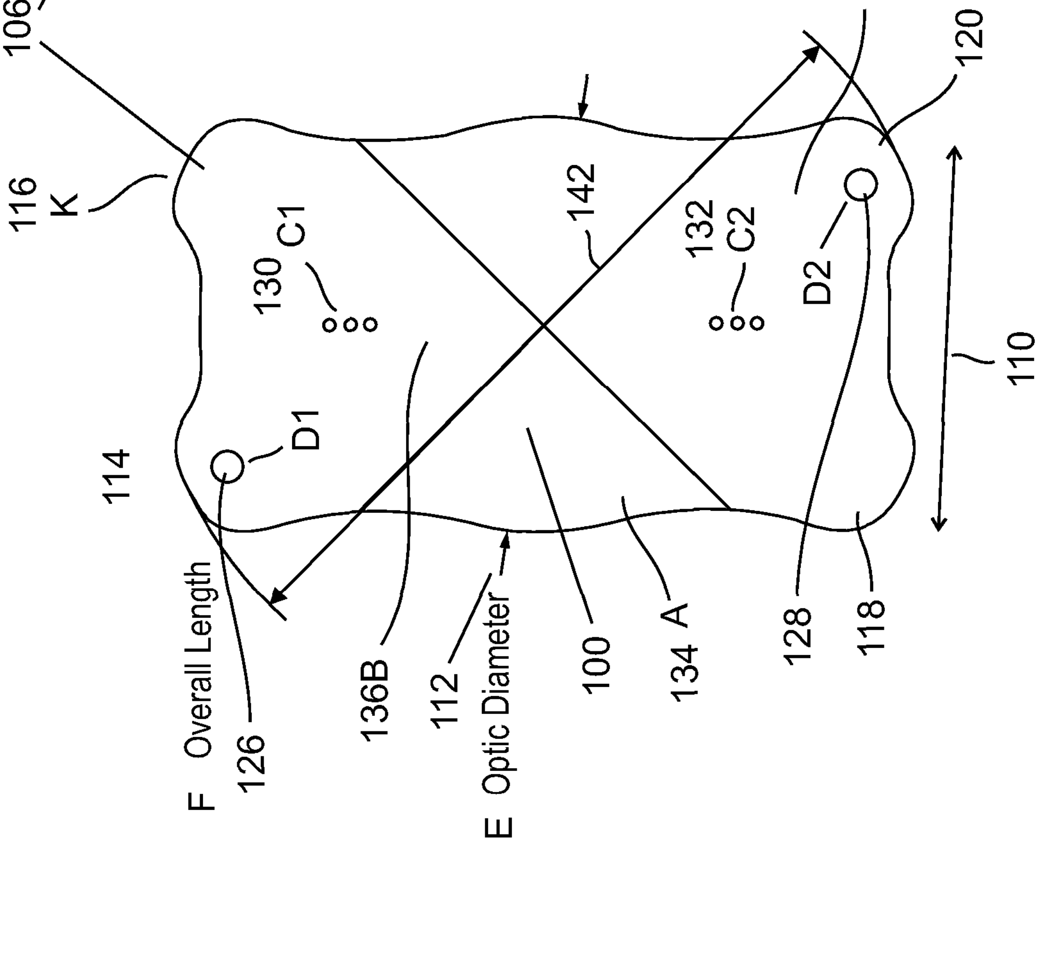
116 K
114
130 C1
D1
142
132 C2
D2
120
110
F Overall Length 126
136B
E Optic Diameter 112
100
134 A
128
118

FIG. 2

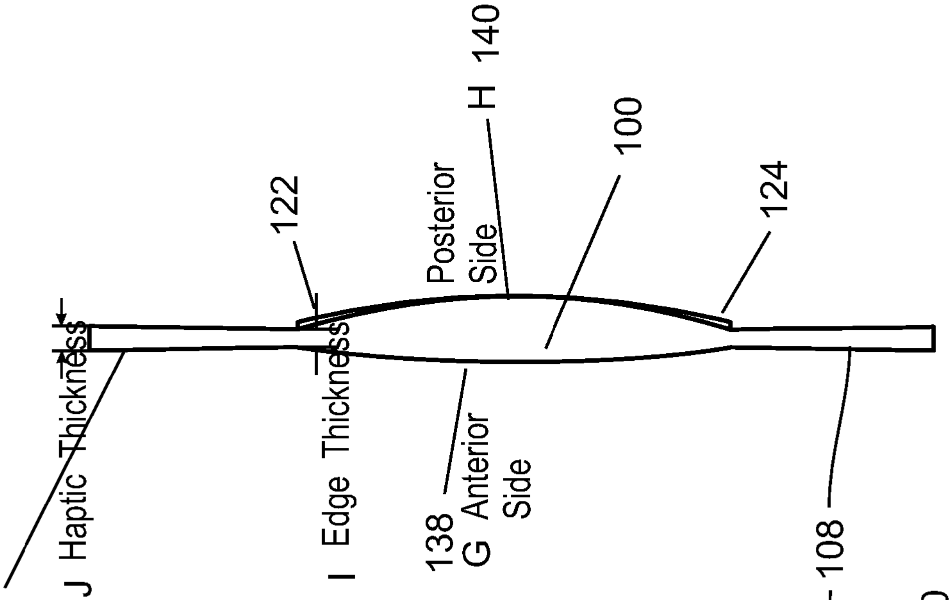

J Haptic Thickness
I Edge Thickness
138 G Anterior Side
Posterior Side
H 140
122
100
124
106
108


FIG. 3

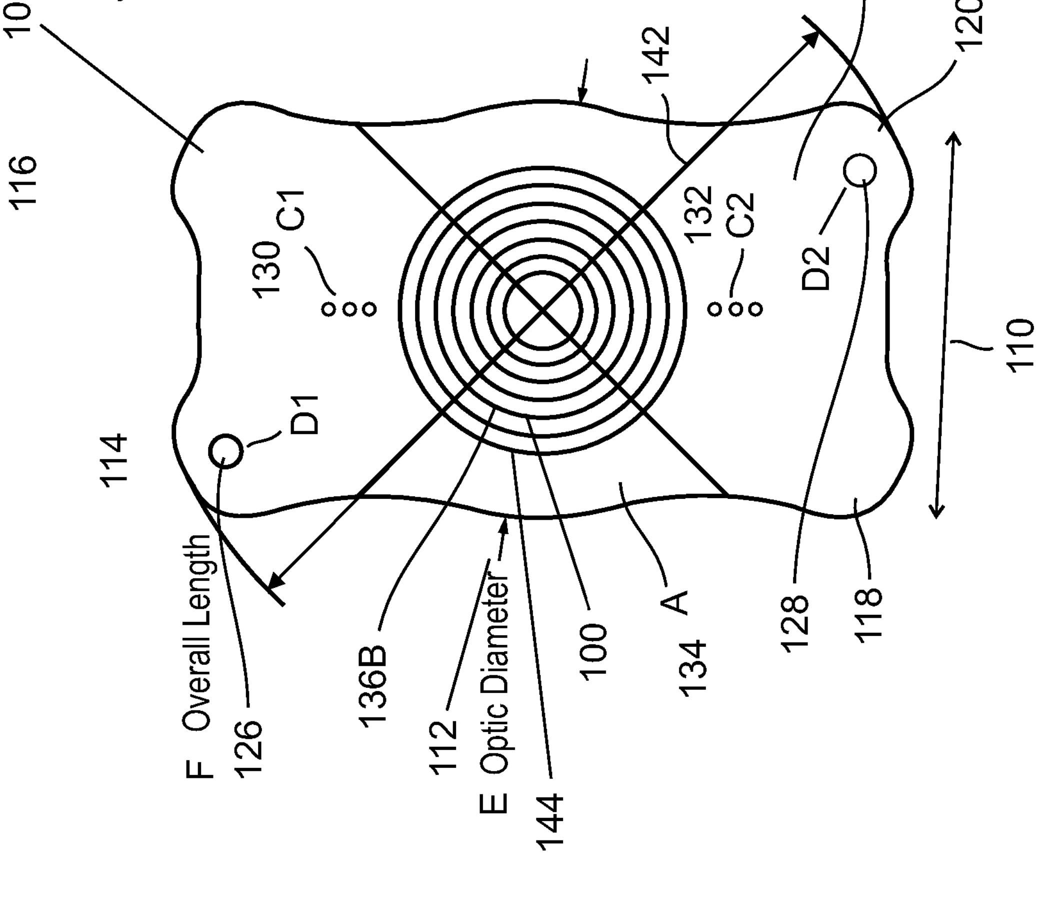

116
114
106
130 C1
132 C2
D1
D2
142
120
110
F Overall Length
126
E Optic Diameter
112
144
136B
100
A
134
128
118


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4881805 A, Cohen
- US 5089023 A
- US 5699142 A, Chun-Shen Lee (Alcon Restor)
- US 534447 A, Swanson
- US 7377641 B, Patricia Ann Piers
- EP 1194797 B1, Acritec AcriLisa
- US 5076684 A
- US 5116111 A, Simpson
- US 5129718 A, Futhey
- US 4637697 A
- US 4641934 A
- US 4655565 A, Freeman
- US 20090323020 A1, Zhao
- US 3722986 A, Tagnon
- US 20080288066 A1
- EP 2111822 A2
- US 5290892 A
- US 4304895 A
- US 4528311 A
- US 7691918 B
- US 3479345 A
- US 20030153512 A
- US 20060276536 A
- US 20070060644 A
- US 20070204412 A
- US 20080200478 A
- US 20100010232 A
- US 20100048901 A
- US 20100087527 A